# EUROPEAN PATENT APPLICATION

(11) **EP 3 656 846 A1**
(43) Date of publication of application: **27.05.2020**
(21) Application number: 18834362.8
(22) Date of filing: 20.07.2018
(51) Int. Cl.: C12N 1/04, C12N 5/079

(54) **METHOD FOR PRESERVING NEURAL TISSUE**

(30) Priority: 20.07.2017 JP 2017141382
(71) Applicant: Riken, Wako-shi, Saitama 351-0198 (JP); Sumitomo Dainippon Pharma Co., Ltd., Osaka-shi Osaka 541-8524 (JP)
(72) Inventor: MANDAI, Michiko, Wako-shi Saitama 351-0198 (JP); TAKAHASHI, Masayo, Wako-shi Saitama 351-0198 (JP); KUWAHARA, Atsushi, Kobe-shi Hyogo 650-0047 (JP); WATARI, Kenji, Kobe-shi Hyogo 650-0047 (JP); MATSUSHITA, Keizo, Osaka-shi, Osaka 5540022 (JP)
(74) Representative: Hoffmann Eitle
(86) International application number: PCT/JP2018/027372
(87) International publication number: WO 2019/017491

(57) **Abstract**

An object of the present invention is to provide a method for preserving neural tissue for several hours to several weeks without freezing, a preservation solution therefor, a transport method for neural tissue in accordance with the method and using the preservation solution, and a composition for transplantation. The preservation method for neural tissue, comprising preserving neural tissue in a preservation solution having a potassium ion concentration of more than 0 mM and less than 115 mM at a preservation temperature of 8°C to 30°C. The preservation solution having a potassium ion concentration of more than 0 mM and less than 115 mM, for preserving neural tissue derived from pluripotent stem cells at a preservation temperature of 8°C to 30°C.

## Description

### Technical Field

The present invention relates to a preservation method for neural tissue, a preservation solution for neural tissue and a transport method for neural tissue; particularly relates to a method for preserving neural tissue for several hours to several weeks without freezing, a preservation solution therefor and a method for transporting neural tissue while preserving. The present invention also relates to a composition for transplantation.

### Background Art

In neural tissues of a living body, a single or a plurality of types of neural cells form a layer structure. One of the neural tissues, retinal tissue, is mainly constituted of 5 types of neuronal cells including photoreceptor cells, bipolar cells, horizontal cells, amacrine cells and ganglion cells, and glial cells, and forms a three-dimensional layer structure. For treating a neurological disease, for example, a retinal degenerative disease, it has been suggested that a transplantation therapy using neural tissue is effective. However, it was difficult to obtain a tissue maintaining a layer structure reflecting the neural tissue of a human living body and a function thereof. Because of this, transplantation therapy was rarely used as a common therapy. Recently, stable production of neural tissue (for example, retinal tissue) has been made possible by differentiation induction from pluripotent stem cells (Non Patent Literatures 1 and 2). Then, as a next step for realizing transplantation therapy using neural tissue, it was required to develop a technique for stably preserving produced neural tissue during the period for conducting quality test or transporting to a transplantation facility.

As a preservation method for neural tissue, cryopreservation has been developed (for example, Patent Literature 1). However, if frozen, quality of the tissue often deteriorates and well-trained technical skill is sometimes required for cryopreservation. In the meantime, it has been reported that liver tissue can be preserved by perfusion using a huge apparatus at a preservation temperature of 22°C (Non Patent Literature 3). However, in the case of neural tissue such as retinal tissue consisting of a single or a plurality of types of neuronal cells, like a layer structure, a suitable preservation method other than freezing is not present. Because of this, establishing a method suitable for preserving the neural tissue for several hours to several weeks is desired.

### Citation List

### Patent Literature

Patent Literature 1: Japanese Patent No. 6012164

### Non Patent Literature

Non Patent Literature 1: Cell Stem Cell, 3, 519-32 (2008)
Non Patent Literature 2: Nature Communications, 6, 6286 (2015)
Non Patent Literature 3: Scientific Reports, 5, 9563 (2015)
Non Patent Literature 4: Transplantation, 74, 1414-9 (2002)

### Summary of Invention

### Technical Problem

An object of the present invention is to provide a preservation method for neural tissue for several hours to several weeks without freezing, a preservation solution therefor, a transport method for neural tissue in accordance with the method and using the preservation solution, and a composition for transplantation.

### Solution to Problem

The present inventors conducted intensive studies with a view to attaining the above object. As a result, they found that neural tissue can be preserved while maintaining a function thereof for several days in a preservation solution having a potassium ion concentration of less than 115 mM at a preservation temperature of 8°C to 30°C, and arrived at the present invention.

More specifically, the present invention relates to the following items.
[1] A preservation method for neural tissue, comprising preserving neural tissue in a preservation solution having a potassium ion concentration of more than 0 mM and less than 115 mM at a preservation temperature of 8°C to 30°C.
[2] The preservation method according to [1], wherein the preservation temperature is 12°C to 22°C.
[3] The preservation method according to [1] or [2], wherein the neural tissue is derived from a human.
[4] The preservation method according to any one of [1] to [3], wherein the neural tissue is a tissue derived from pluripotent stem cells.
[5] The preservation method according to any one of [1] to [4], wherein the neural tissue is a tissue to be subjected to transplantation.
[6] The preservation method according to any one of [1] to [5], wherein the neural tissue is a cell aggregate having a layer structure.
[7] The preservation method according to [6], wherein the neural tissue is a cell aggregate having a neuroepithelial structure.
[8] The preservation method according to [7], wherein the neuroepithelial structure is a continuous epithelium structure present on a surface of the cell aggregate and/or a rosette structure present on a surface of or inside the cell aggregate.
[9] The preservation method according to any one of [1] to [8], wherein the neural tissue includes one or more neural tissues selected from the group consisting of forebrain tissue, midbrain tissue and retinal tissue.
[10] The preservation method according to any one of [1] to [9], for preservation for a period of 14 days or less.
[11] The preservation method according to any one of [1] to [9], for preservation for a period of 5 days or less.
[12] The preservation method according to any one of [1] to [11], wherein the preservation solution is an aqueous solution containing a buffering agent and having a buffering action to control pH within the range of 6.0 to 8.6.
[13] The preservation method according to any one of [1] to [12], wherein the preservation solution is the aqueous solution further containing a glycosaminoglycan.
[14] The preservation method according to [13], wherein the concentration of the glycosaminoglycan is 0.1% (w/v) or more and 10% (w/v) or less.
[15] The preservation method according to [13] or [14], wherein the glycosaminoglycan includes one or more glycosaminoglycans selected from the group consisting of chondroitin sulfate and hyaluronic acid.
[16] A preservation solution having a potassium ion concentration of more than 0 mM and less than 115 mM, for preserving neural tissue derived from pluripotent stem cells at a preservation temperature of 8°C to 30°C.
[17] The preservation solution according to [16], wherein the preservation temperature is 12°C to 22°C.
[18] The preservation solution according to [16] or [17], wherein the neural tissue is a cell aggregate having a layer structure.
[19] The preservation solution according to [18], wherein the neural tissue is a cell aggregate having a neuroepithelial structure.
[20] The preservation solution according to [19], wherein the neuroepithelial structure is a continuous epithelium structure present on a surface of the cell aggregate and/or a rosette structure present on a surface of or inside the cell aggregate.
[21] A transport method for neural tissue, comprising transporting neural tissue in a preservation solution having a potassium ion concentration of more than 0 mM and less than 115 mM at a preservation temperature of 8°C to 30°C.
[22] The transport method according to [21], wherein the preservation temperature is 12°C to 22°C.
[23] The transport method according to [21] or [22], wherein the neural tissue is a tissue derived from human pluripotent stem cells.
[24] The transport method according to any one of [21] to [23], wherein the neural tissue is a tissue to be subjected to transplantation.
[25] The transport method according to any one of [21] to [24], wherein the neural tissue is a cell aggregate having a layer structure.
[26] The transport method according to [25], wherein the neural tissue is a cell aggregate having a neuroepithelial structure.
[27] The transport method according to [26], wherein the neuroepithelial structure is a continuous epithelium structure present on a surface of the cell aggregate and/or a rosette structure present on a surface of or inside the cell aggregate.
[28] The transport method according to any one of [21] to [27], wherein the neural tissue is one or more neural tissues selected from the group consisting of forebrain tissue, midbrain tissue and retinal tissue.
[29] A composition for transplantation at a temperature of 12°C to 22°C, comprising an aqueous solution having a potassium ion concentration of more than 0 mM and less than 115 mM and pH controlled by a buffering agent within the range of 6.0 to 8.6 and a human neural tissue derived from pluripotent stem cells.
[30] The composition according to [29], wherein the neural tissue is a cell aggregate having a neuroepithelial structure.
[31] The composition according to [30], wherein the neuroepithelial structure is a continuous epithelium structure present on a surface of the cell aggregate and/or a rosette structure present on a surface of or inside the cell aggregate.
[32] The composition according to any one of [29] to [31], wherein the neural tissue is one or more neural tissues selected from the group consisting of forebrain tissue, midbrain tissue and retinal tissue.
[33] The composition according to any one of [29] to [32], wherein the aqueous solution is the aqueous solution further containing a glycosaminoglycan of 0.1% (w/v) or more and 10% (w/v) or less.

### Advantageous Effects of Invention

According to the present invention, neural tissue taken out of a living body or obtained through differentiation induction from pluripotent stem cells can be preserved for several weeks without freezing until the neural tissue is subjected to transplantation, more specifically, during the period of conducting quality control before subjecting it to transplantation and the period for transporting it. Owing to this, treatment for a disease caused by damage in neural tissue or the damage state in neural tissue by transplantation therapy becomes more feasible.

### Brief Description of Drawings

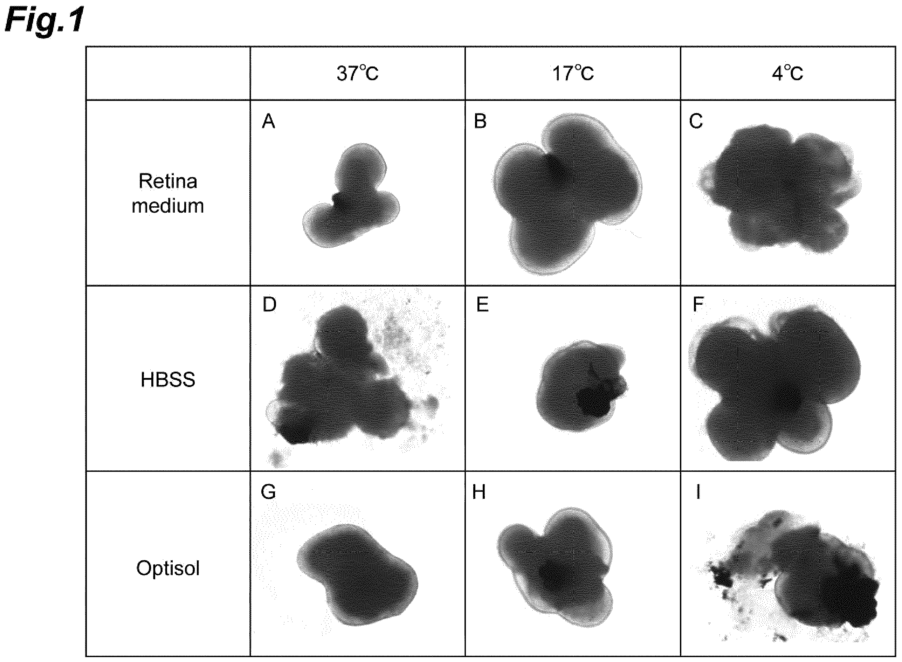

[Figure 1] Figure 1 shows the bright-field images of cell aggregates containing retinal tissue prepared from human iPS cells after the cell aggregates were preserved in the various preservation conditions, and subjected to recovery culture at 37°C for 7 days.

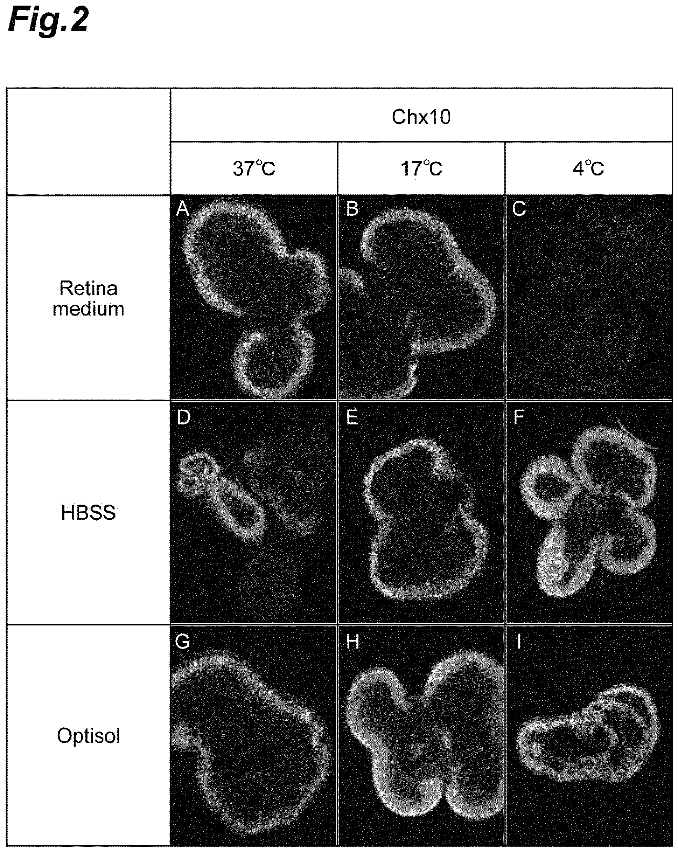

[Figure 2] Figure 2 shows comparative results of immunohistochemical staining of Chx10 expressed in cell aggregates containing retinal tissue prepared from human iPS cells after the cell aggregates were preserved in the various preservation conditions and subjected to recovery culture at 37°C for 7 days.

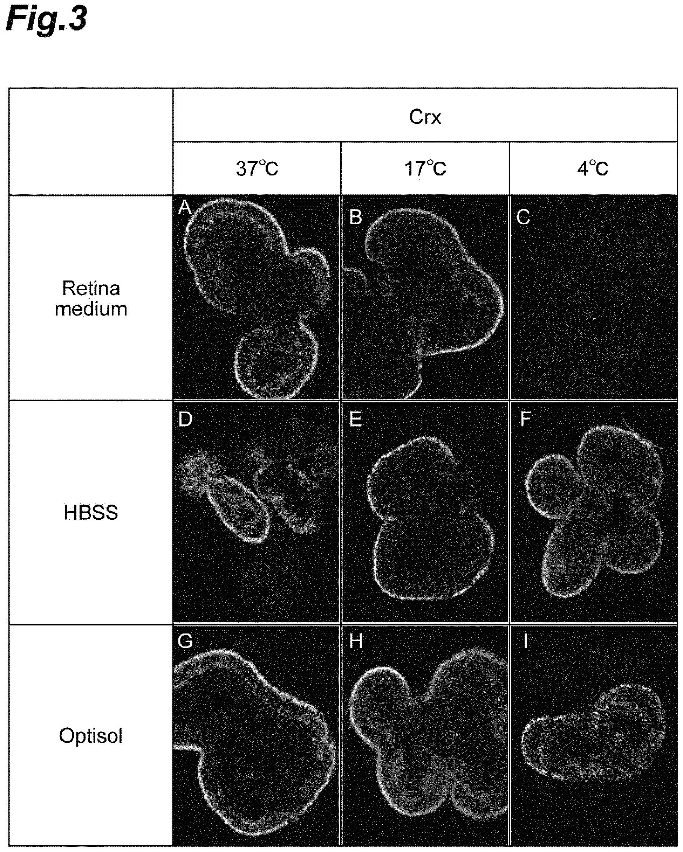

[Figure 3] Figure 3 shows comparative results of immunohistochemical staining of Crx expressed in cell aggregates containing retinal tissue prepared from human iPS cells after the cell aggregates were preserved in the various preservation conditions and subjected to recovery culture at 37°C for 7 days.

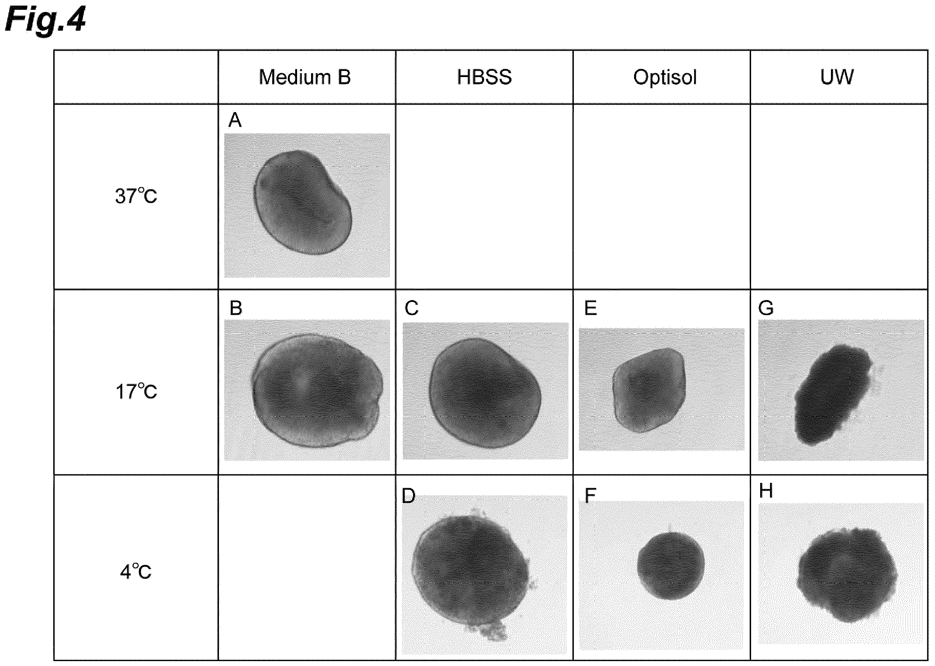

[Figure 4] Figure 4 shows the bright-field images of cell aggregates containing retinal tissue prepared from human ES cells after the aggregates were preserved in the various preservation conditions and subjected to recovery culture at 37°C for 7 days.

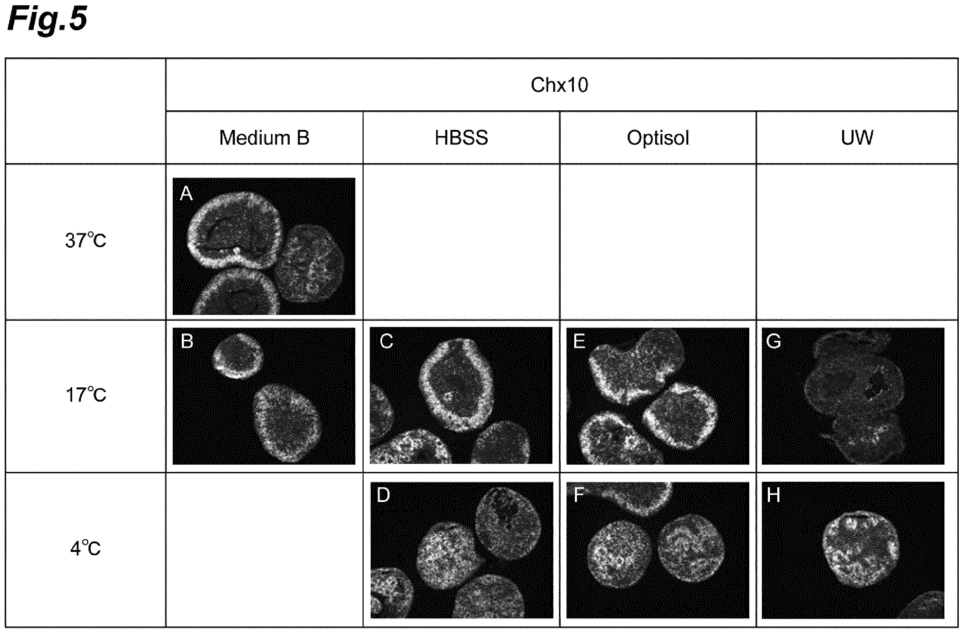

[Figure 5] Figure 5 shows comparative results of immunohistochemical staining of Chx10 expressed in cell aggregates containing retinal tissue prepared from human ES cells after the cell aggregates were preserved in the various preservation conditions and subjected to recovery culture at 37°C for 7 days.

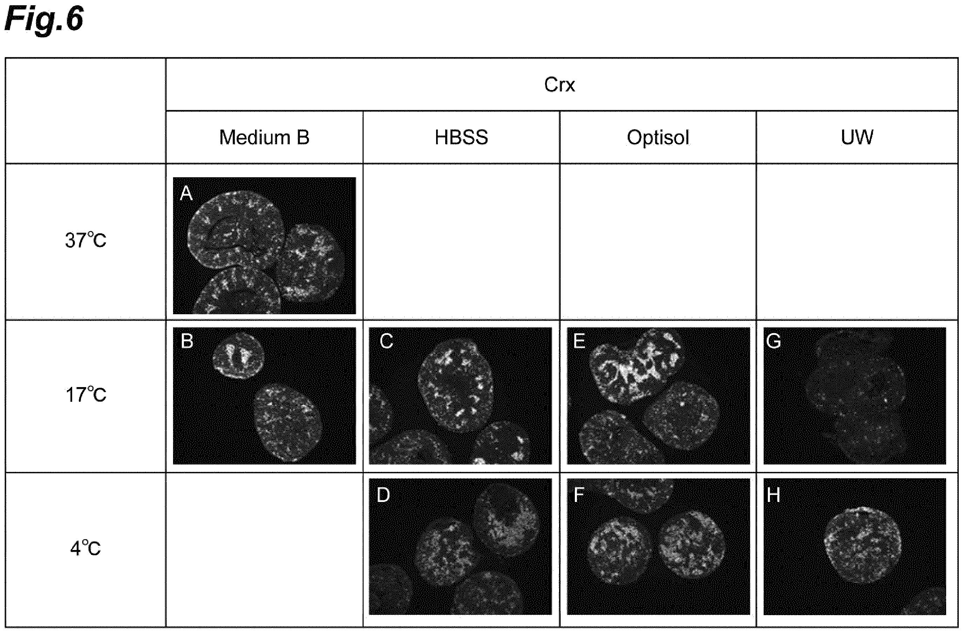

[Figure 6] Figure 6 shows comparative results of immunohistochemical staining of Crx expressed in cell aggregates containing retinal tissue prepared from human ES cells after the cell aggregates were preserved in the various preservation conditions and subjected to recovery culture at 37°C for 7 days.

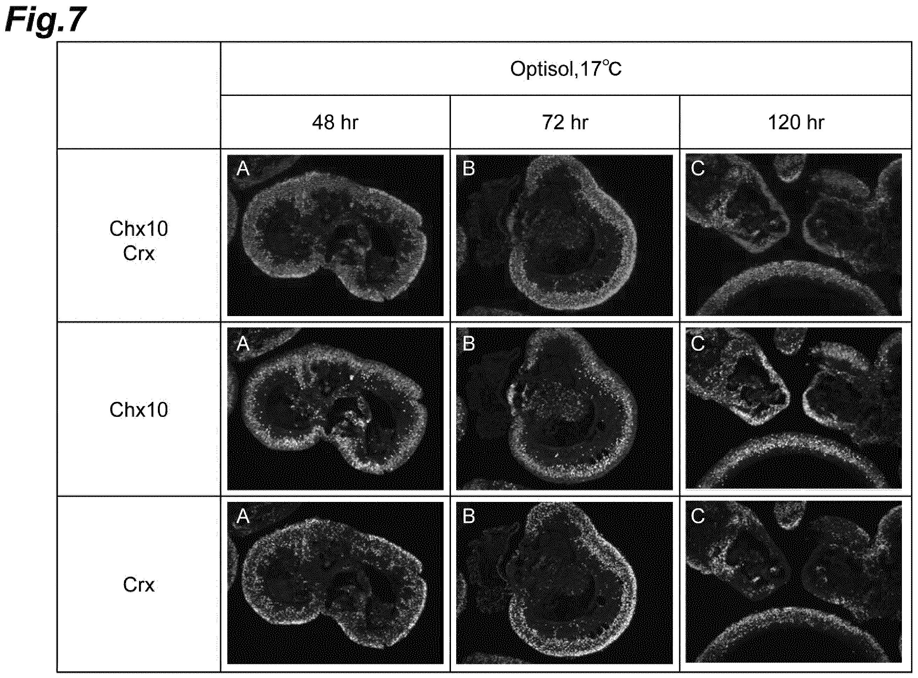

[Figure 7] Figure 7 shows comparative results of immunohistochemical staining of various proteins expressed in cell aggregates containing retinal tissue prepared from human iPS cells after the cell aggregates were preserved in the various preservation conditions and subjected to recovery culture at 37°C for 7 days. The top stage shows Chx10 and Crx, middle stage Chx10 and the bottom stage Crx.

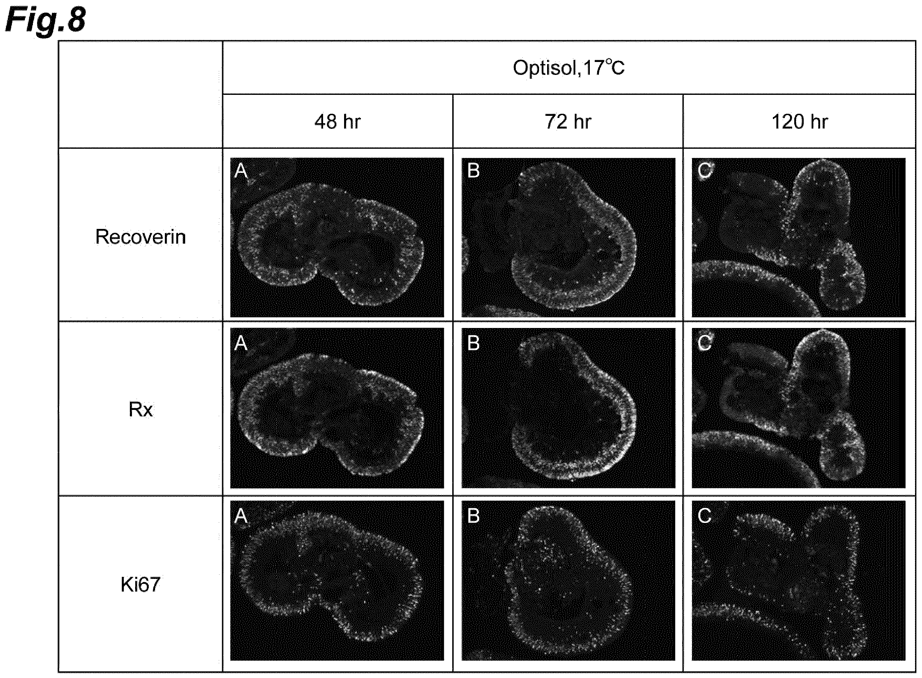

[Figure 8] Figure 8 shows comparative results of immunohistochemical staining of various proteins expressed in cell aggregates containing retinal tissue prepared from human iPS cells, after the cell aggregates were preserved in the various preservation conditions and subjected to recovery culture at 37°C for 7 days. The top stage shows Recoverin, the middle stage Rx and the bottom stage Ki67.

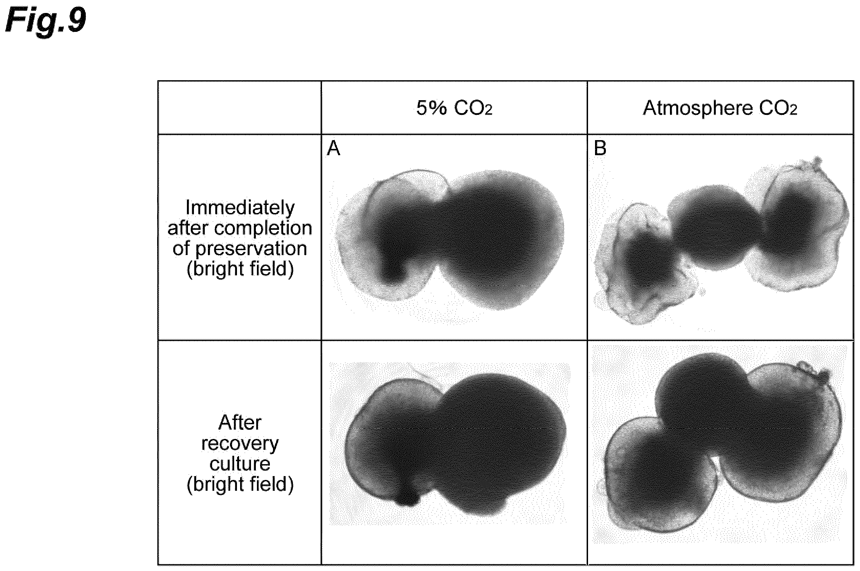

[Figure 9] Figure 9 shows the bright-field images of cell aggregates containing retinal tissue prepared from human iPS cells and observed immediately after completion of preservation in the various preservation conditions (preservation conditions A and B) and after subjected to recovery culture at 37°C for 7 days.

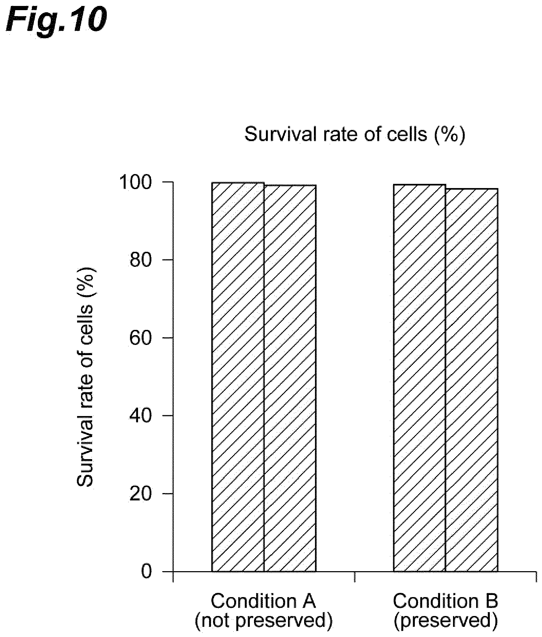

[Figure 10] Figure 10 shows the survival rate of cells in a cell aggregate containing retinal tissue prepared from human iPS cells measured before and after preservation thereof.

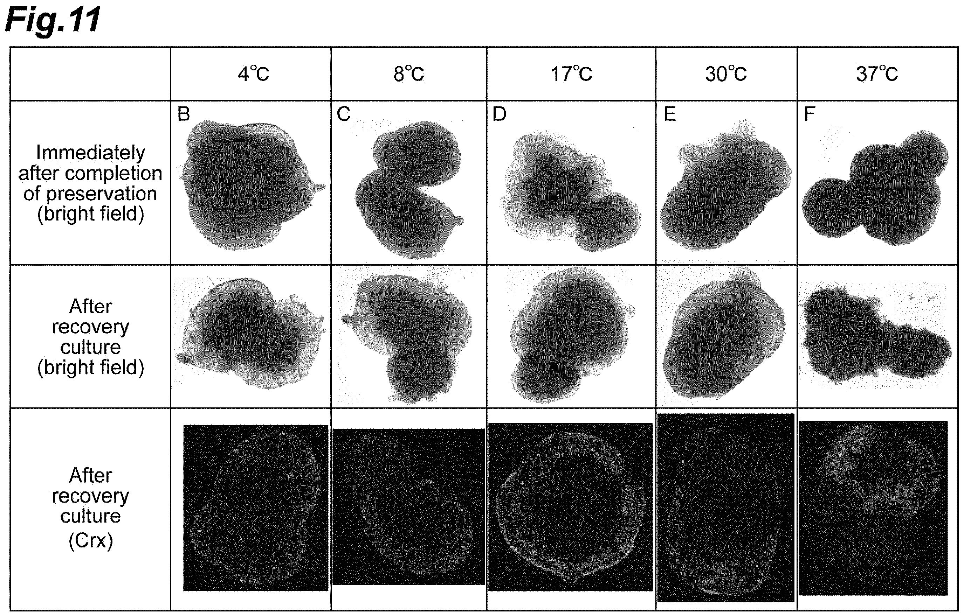

[Figure 11] The top stage of Figure 11 shows the bright-field images of cell aggregates containing retinal tissue prepared from human iPS cells and preserved in the various preservation conditions (preservation conditions B to F), immediately after the preservation; the middle stage shows the bright-field images thereof obtained by further subjecting the aggregates to recovery culture at 37°C for 7 days; and the bottom stage shows comparative results of immunohistochemical staining of Crx expressed in the cell aggregates after the recovery culture.

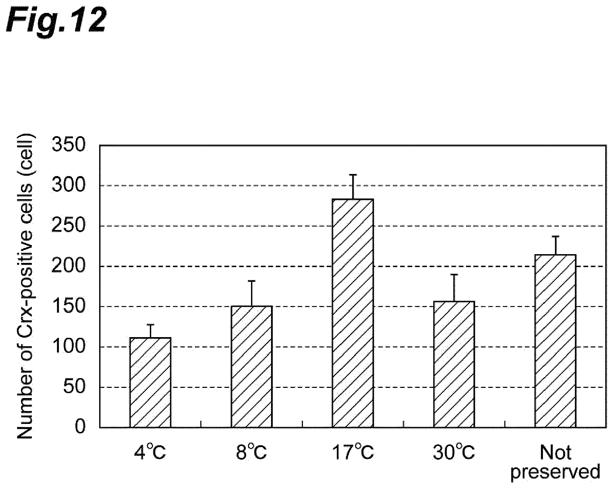

[Figure 12] Figure 12 is a graph showing the number of Crx-positive cells determined based on the results shown in Figure 11.

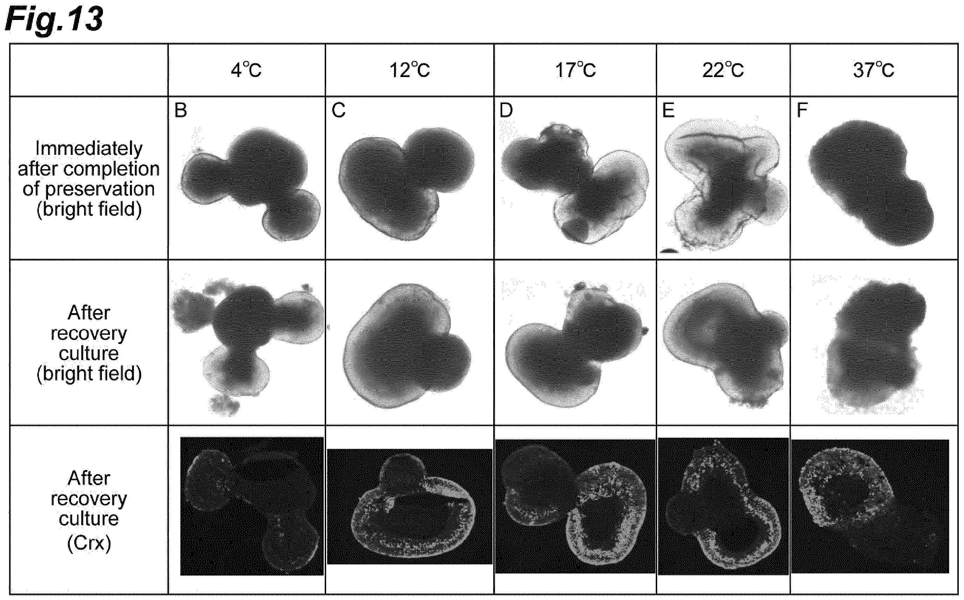

[Figure 13] The top stage of Figure 13 shows the bright-field images of cell aggregates containing retinal tissue prepared from human iPS cells and preserved in the various preservation conditions (preservation conditions B to F), immediately after the preservation; the middle stage shows the bright-field images thereof obtained by further subjecting the aggregates to recovery culture at 37°C for 7 days; and the bottom stage shows comparative results of immunohistochemical staining of Crx expressed in the cell aggregates after the recovery culture.

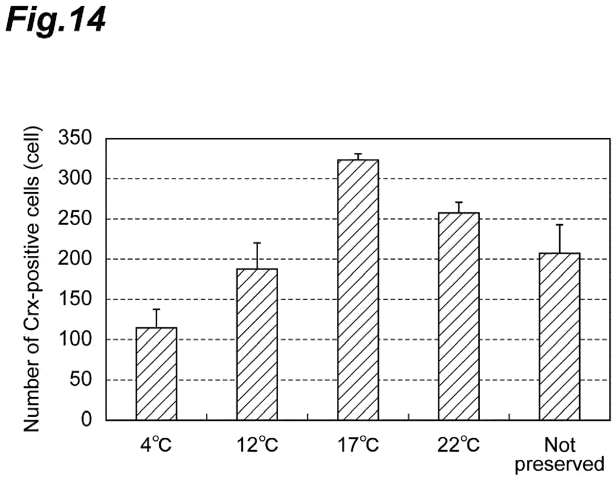

[Figure 14] Figure 14 is a graph showing the number of Crx-positive cells determined based on the immunohistochemical staining results shown in Figure 11.

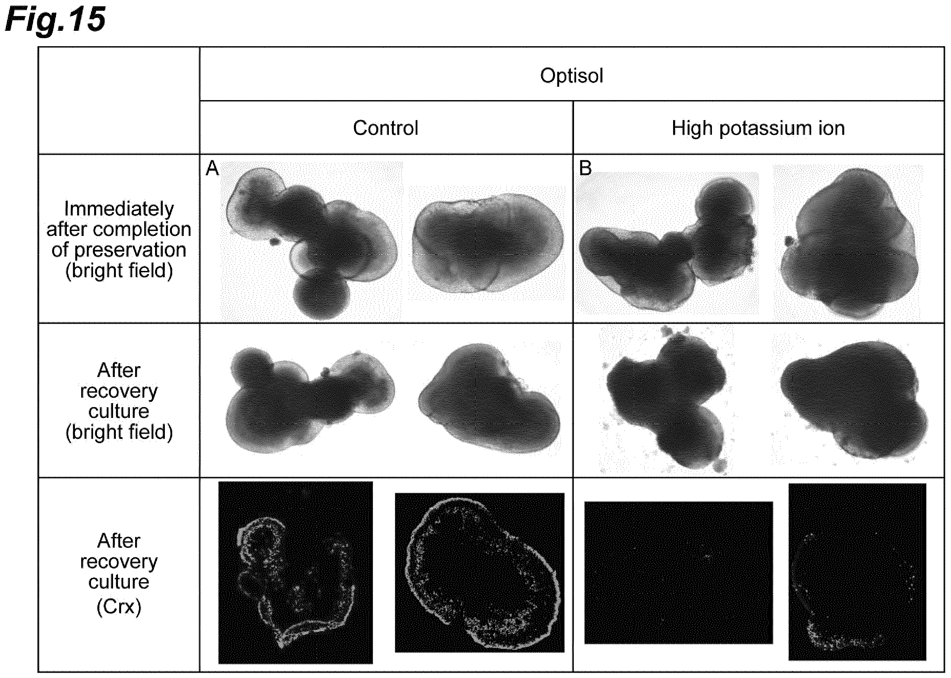

[Figure 15] The top stage of Figure 15 shows the bright-field images of cell aggregates containing retinal tissue prepared from human iPS cells and preserved in the various preservation conditions (preservation conditions A and B), immediately after the preservation; the middle stage shows the bright-field images thereof obtained by further subjecting the aggregates to recovery culture at 37°C for 7 days; and the bottom stage shows comparative results of immunohistochemical staining of Crx expressed in the cell aggregates after the recovery culture.

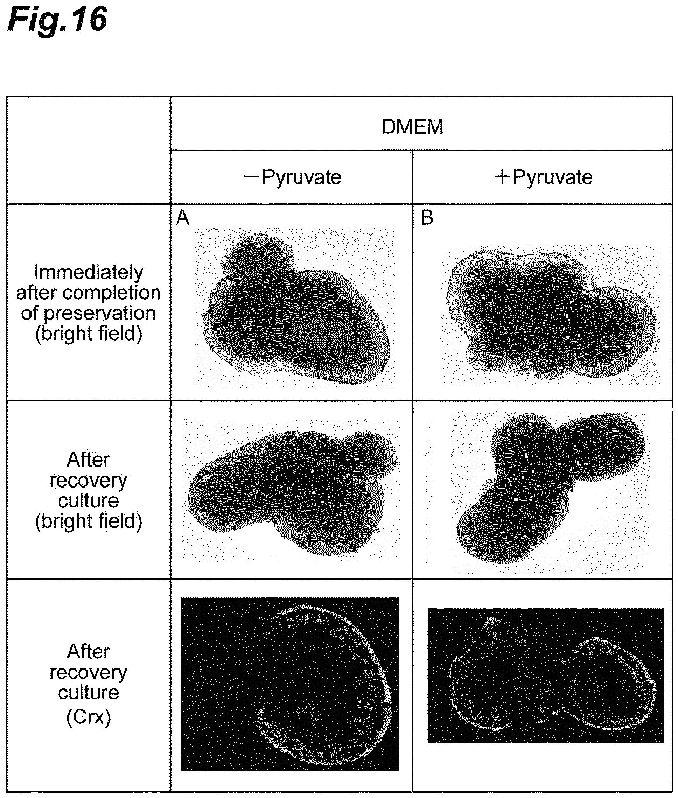

[Figure 16] The top stage of Figure 16 shows the bright-field images of cell aggregates containing retinal tissue prepared from human iPS cells and preserved in the various preservation conditions (preservation conditions A and B), immediately after the preservation; the middle stage shows the bright-field images thereof obtained by further subjecting the aggregates to recovery culture at 37°C for 7 days; and the bottom stage shows comparative results of immunohistochemical staining of Crx expressed in the cell aggregates after recovery culture.

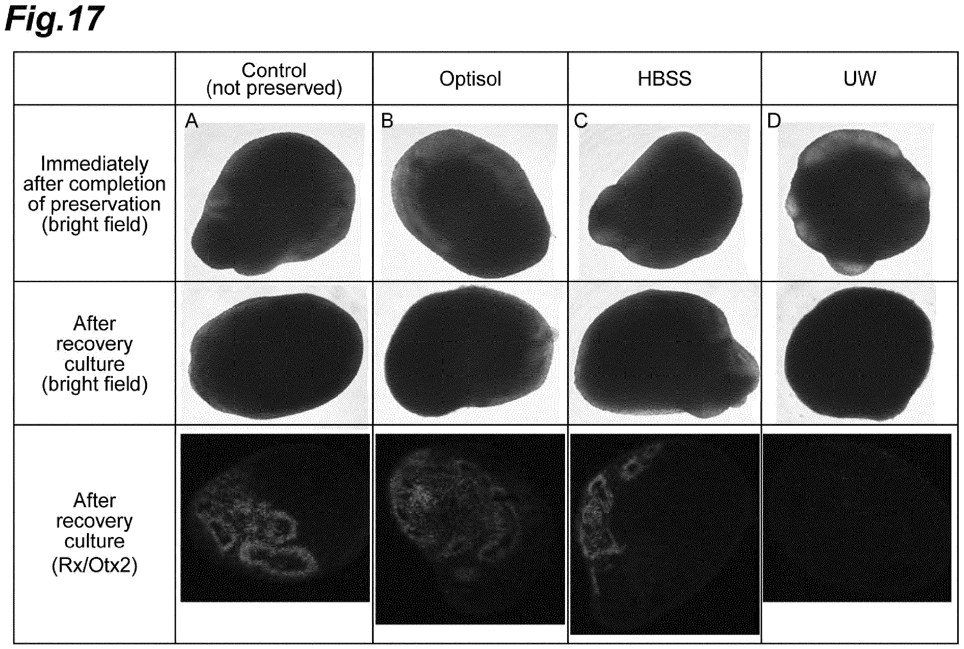

[Figure 17] The top stage of Figure 17 shows the bright-field images of cell aggregates containing forebrain tissue or midbrain tissue prepared from human iPS cells and preserved in the various preservation conditions (preservation conditions A to D), immediately after the preservation; the middle stage shows the bright-field images thereof obtained by further subjecting the aggregates to recovery culture at 37°C for 7 days; and the bottom stage shows comparative results of immunohistochemical staining of Rx and Otx2 expressed in the cell aggregates after the recovery culture.

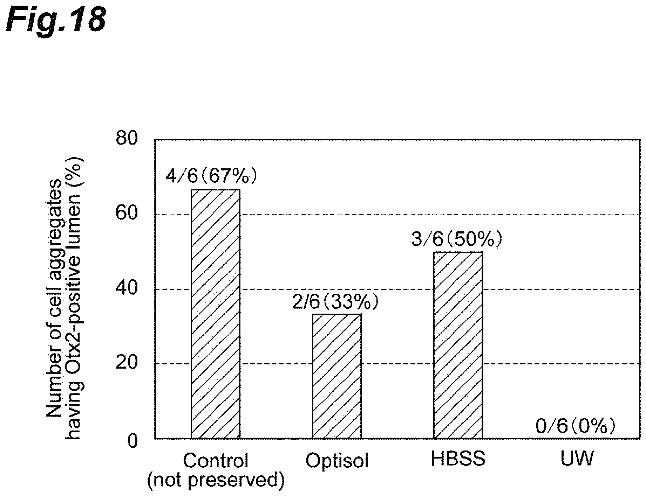

[Figure 18] Figure 18 is a graph showing the ratio of the number of cell aggregates in which a lumen structure formed of Otx2-positive cells was observed, relative to the whole number of cell aggregates versus individual preservation conditions based on the results of the immunohistochemical staining shown in Figure 17.

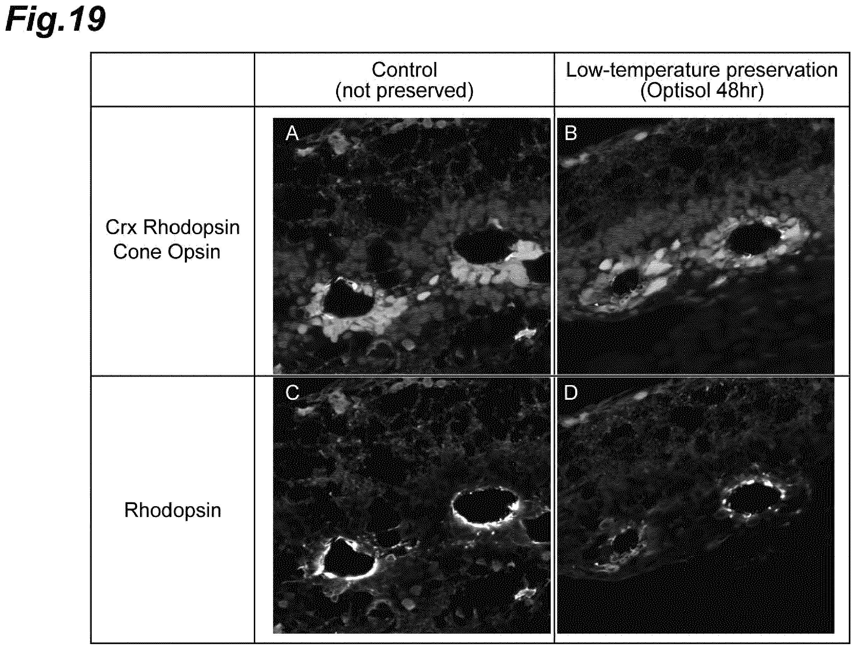

[Figure 19] Figure 19 shows immunohistochemically stained images of frozen sections of a nude rat retina, which is obtained after cell aggregates having retinal tissue prepared from human ES cells were exposed to48-hours preservation (represented by B and D) and without preservation (represented by A and C) and then transplanted to the subretina of a retinal degenerative nude rat.

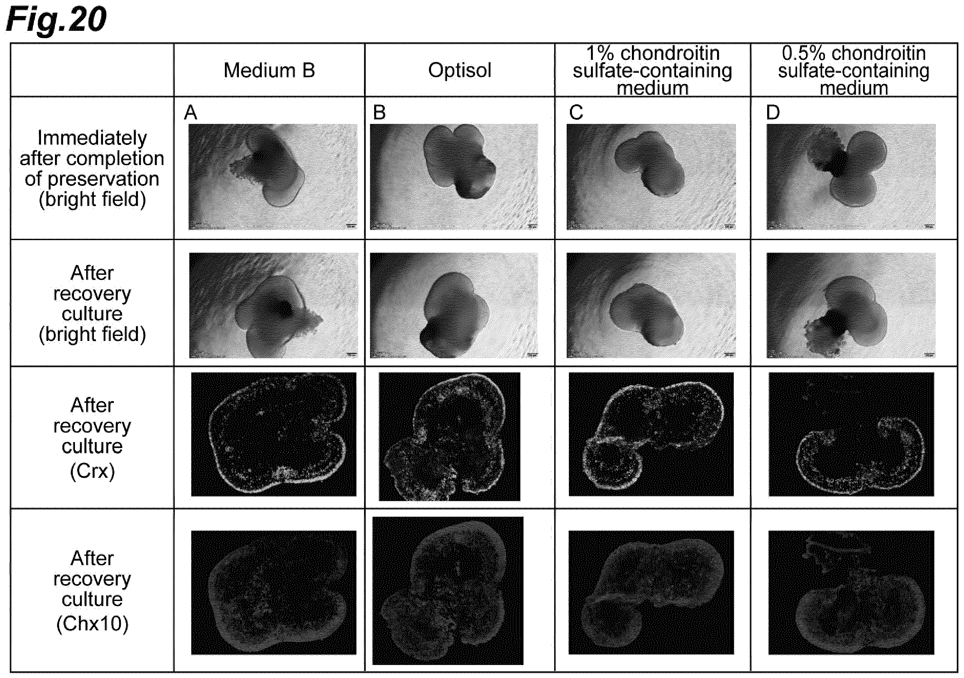

[Figure 20] The top stage of Figure 20 shows the bright-field images of cell aggregates containing retinal tissue prepared from human iPS cells and preserved in the various preservation conditions (preservation conditions A to D), immediately after the preservation; the second stage from the top shows the bright-field images thereof obtained by further subjecting the aggregates to recovery culture at 37°C for 7 days; the second stage from the bottom shows comparative results of immunohistochemical staining of photoreceptor precursor cell marker Crx in the cell aggregates after recovery culture; and the bottom stage shows comparative results of immunohistochemical staining of photoreceptor precursor cell marker Chx10 in the cell aggregates after recovery culture.

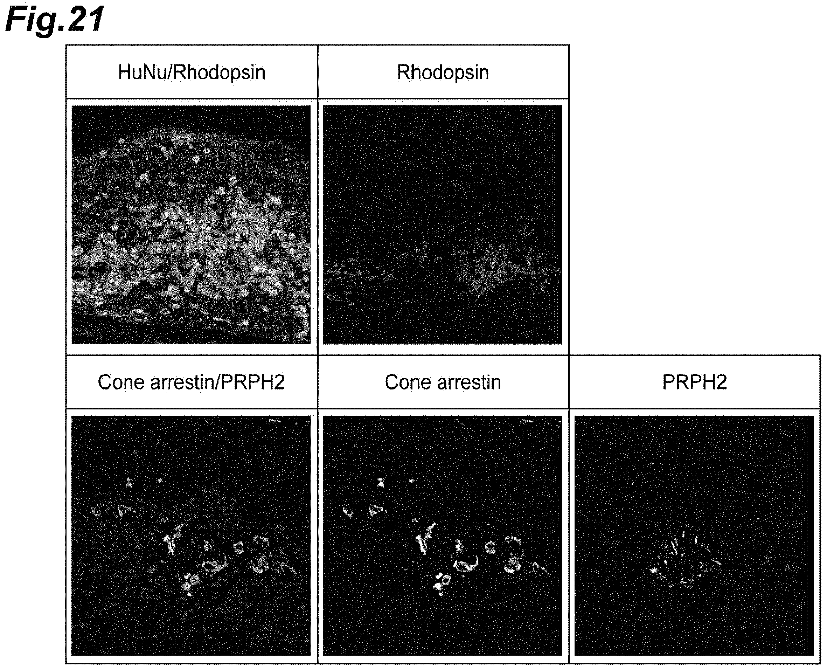

[Figure 21] Figure 21 shows immunohistochemically stained images of frozen sections of a nude rat retina, which is obtained after cell aggregates having retinal tissue prepared from human iPS cells were exposed to an Optisol solution at temperature of 17°C for 96 hours and then transplanted to the subretina of a retinal degenerative nude rat.

### Description of Embodiments

The preservation method for neural tissue of the present invention comprises preserving neural tissue in a preservation solution having a potassium ion concentration of more than 0 mM and less than 115 mM at a preservation temperature of 8°C to 30°C.

In the specification, the "tissue" refers to a structure of cell population (also referred to as a "cell aggregate"), that is, a structure formed by three-dimensionally arranging homologous cells same in form and nature or heterologous cells different in form and nature in accordance with a predetermined pattern. The tissue, which are collected from a living body or artificially prepared from stem cells, is maintained *ex vivo* in an isolated state.

In the present invention, in view of developmental biology, the cells are morphologically distinguished into epithelial cells and mesenchymal cells. The epithelial cells have a polarity in the apical-basal direction. The apical side is often a side having void spaces; whereas the basal side has a basement membrane and is in contact with the extracellular matrix (extra-cellular matrix). The epithelial cells can mutually and firmly join via adherence junction or tight junction on the apical side to form epithelium. The epithelium, which is also referred to as epithelial sheet, includes single layer squamous epithelium, single layer columnar epithelium and stratified squamous epithelium. The mesenchymal cells, which are cells having polarity in apical-basal direction but poor and having little contribution to adherence junction and/or tight junction, rarely form sheet. The mesenchymal cells in a living body are mostly present sporadically like stars in extracellular matrix.

In the specification, the "neural tissue" refers to a tissue constituted of neuronal cells and having a three-dimensional structure in which a single or a plurality of types of neuronal cells are arranged like a layer. Typical examples of the neural tissue include forebrain tissue, midbrain tissue and retinal tissue, as well as cerebrum, cerebellum, diencephalon, hindbrain, telencephalon and spinal cord tissues. A structure in which at least a plurality of types of cells and/or precursor cells constituting these tissues are three-dimensionally arranged like a layer, and a combination of these tissues are also included in the neural tissue. The neural tissue can be confirmed by observation by an optical microscope (for example, bright-field microscope, phase-contrast microscope, differential interference microscope, Hoffman interference microscope, stereo microscope) based on expression of a neural tissue marker such as PSA-NCAM and N-cadherin used as an index.

The neural tissue may sometimes form a structure called a neuroepithelial structure. The neuroepithelial structure refers to form constituted of neuronal cells and having a three-dimensional structure in which a single or a plurality of types of neuronal cells are arranged like a layer. In an embodiment, the neuroepithelial structure has a polarity in the apical-basal direction.

In some embodiments, the neuroepithelial structure may be present so as to cover the surface of a cell aggregate. In other embodiments, the neuroepithelial structure can be also formed inside a cell aggregate.

In some embodiments, the neuroepithelial structure may be present as a continuous epithelium structure (described later) on the surface of a cell aggregate or as a rosette structure (described later, also referred to as nerve rosette and lumen structure) on the surface of a cell aggregate or inside a cell aggregate.

The abundance of the neuroepithelial structure present within a cell aggregate can be evaluated by bright-field observation using an optical microscope.

In the present invention, the "neural cells" refer to cells in the ectoderm-derived tissue except epidermal cells (e.g., epidermis, hair, nail, sebaceous glands). More specifically, neural precursor cells, neuron (neuronal cells), glia, neural stem cells, neuron precursor cells and glial precursor cells are included. Examples of the neural cells include retinal cells constituting retinal tissue (described later), retinal precursor cells, retinal layer specific neuronal cells, neural retinal cells and retinal pigment epithelial cells.

The neuronal cells (or neuron) are functional cells forming a neural circuit and contributing to signal transduction, and can be identified based on expression of a juvenile neuronal cell marker such as TuJ1, Dcx and HuC/D and/or a mature neuronal cell marker such as Map2 and NeuN.

Examples of the glia includes astrocytes, oligodendrocytes and Muller glia. An example of a marker for astrocytes is GFAP; an example of a marker for oligodendrocytes is 04; an example of a marker for Muller glia is CRALBP.

Neural stem cells are the cells having differentiation potency (pluripotency) into neuronal cells and glial cells, and proliferation potency (sometimes referred to as self-replicating ability) while maintaining pluripotency. Examples of a marker for the neural stem cells include Nestin, Sox2, Musashi, Hes family and CD133. These markers are markers for whole precursor cells and not considered as specific markers for neural stem cells. The number of neural stem cells can be evaluated by, e.g., neurosphere assay or clonal assay.

The neuron precursor cells are cells having proliferation potency and producing neuronal cells and not producing glial cells. Examples of a marker for neuron precursor cells include Tbr2 and Tα1. Alternatively, juvenile neuronal cell marker (TuJ1, Dcx, HuC/D)-positive cells and proliferation marker (Ki67, pH3, MCM)-positive cells can be identified as neuron precursor cells.

The glial precursor cells refer to cells having proliferation potency, differentiating into glial cells and not differentiating into neuronal cells.

The neural precursor cells are an aggregation of precursor cells including neural stem cells, neuron precursor cells and glial precursor cells and have proliferation potency and differentiation potency into neurons and glial cells. The neural precursor cells can be identified by using, e.g., Nestin, GLAST, Sox2, Sox1, Musashi and Pax6, as a marker. Alternatively, neural cell marker-positive cells and proliferation marker (Ki67, pH3, MCM)-positive cells can be identified as the neural precursor cells.

In the specification, the "brain tissue" refers to a front region of the neural tissue along the anteroposterior axis of the neural tissue. The brain of a living body is developed from the brain tissue in the fetal period. The brain tissue is divided into forebrain, midbrain and hindbrain along the anteroposterior axis.

In the specification, the "forebrain tissue" refers to a front region of the brain tissue along the anteroposterior axis. The forebrain tissue includes telencephalon tissue, diencephalon tissue, cerebrum tissue, diencephalon tissue, cerebral cortex tissue, hypothalamus tissue and thalamus, which are in an advanced stage of development. In the specification, tissues developed from the aforementioned tissues are also included in the "forebrain tissue". For example, the retinal tissue, since it is developed from the diencephalon tissue, is included in the forebrain tissue. In some embodiments, the forebrain tissue on a culture dish sometimes forms a neuroepithelial structure; whereas, in some embodiments, the forebrain tissue on a culture dish sometimes has a continuous epithelium structure and/or a rosette structure.

In the specification, the "midbrain tissue" refers to the region of the brain tissue posterior to the forebrain and anterior to the hindbrain along the anteroposterior axis. The midbrain tissue includes, e.g., tegmentum, superior colliculus, inferior colliculus, substantia nigra, red nucleus and cerebral crus. In some embodiments, the midbrain tissue on a culture dish sometimes forms a neuroepithelial structure; whereas, in some embodiments, the midbrain tissue on a culture dish sometimes has a continuous epithelium structure and/or a rosette structure.

Which region of the brain tissue a target tissue belongs to can be checked based on expression of a marker gene described in the literature (Shiraishi et al. Development 2017).

Examples of the marker gene include, but not limited to, Six3 (anterior of the forebrain), FoxG1 (telencephalon in the anterior of the forebrain), Rx (posterior of the telencephalon in the forebrain), Otx2 (part of the forebrain and midbrain), Otx2-positive and Rx-negative genes (in the region in the forebrain or midbrain except retinal tissue) and Irx3 (midbrain or hindbrain).

In the specification, the "retinal tissue" refers to a tissue having a three-dimensional structure in which a single or (at least) a plurality of types of neuronal cells, such as photoreceptor cells, horizontal cells, bipolar cells, amacrine cells, retinal ganglion cells, these precursor cells or retinal precursor cells, are arranged like a layer. Which layer the individual cells constitute can be checked by a method known to those skilled in the art, for example, by determining the presence or absence of expression or expression level of a cell marker (e.g., Chx10 (retinal precursor cells or bipolar cell), L7 (bipolar cell), TuJl (retinal ganglion cells), Brn3 (retinal ganglion cells), Calretinin (amacrine cells), Calbindin (horizontal cell), Recoverin (photoreceptor), Rhodopsin (photoreceptor), RPE65 (retinal pigment epithelial cells) and Mitf (retinal pigment epithelial cells)).

In the specification, the "retinal layer" refers to individual layers constituting the retina, and specific examples thereof include a pigmented layer of retina and a neural retinal layer. Examples of the neural retina layer include external limiting membrane, photoreceptor layer (outer nuclear layer), outer plexiform layer, inner nuclear layer, inner plexiform layer, ganglion cell layer, nerve fiber layer and inner limiting membrane.

In the specification, the "retinal precursor cells" refer to precursor cells capable of differentiating into any one of the mature retinal cells such as photoreceptor cells, horizontal cells, bipolar cells, amacrine cells, retinal ganglion cells and retinal pigment epithelial cells. Examples of the marker for the retinal precursor cells include Rx (also referred to as Rax), PAX6 and ChxlO.

In the specification, the "stem cells" refer to undifferentiated cells having differentiation potency and proliferation potency (particularly self-replicating ability). In the stem cells, subgroups of pluripotent stem cells, multipotent stem cells and unipotent stem cells, are included. The pluripotent stem cells refer to stem cells that can be cultured *in vitro* and having an ability (pluripotency) to differentiate into three germ layers (ectoderm, mesoderm, endoderm) and/or all cell lineages belonging to the extraembryonic tissue. The multipotent stem cells refer to cells having an ability to differentiate into a plurality of tissues or cells, although the definition is not applied to all of them. The unipotent stem cells refer to stem cells having an ability to differentiate into a predetermined tissue or cells.

The "pluripotent stem cells" can be induced from, e.g., a fertilized egg, a cloned embryo, germline stem cells, tissue stem cells and somatic cells. Examples of the pluripotent stem cells include embryonic stem cells (ES cells), embryonic germ cells (EG cells) and induced pluripotent stem cells (iPS cells). Muse cells (Multi-lineage differentiating stress enduring cells) obtained from the mesenchymal stem cells (MSC) and GS cells prepared from germ cells (for example, testis) are included in the pluripotent stem cells.

Human embryonic stem cells were established in 1998 and have been used also for regenerative medicine. The embryonic stem cells can be produced by culturing inner cell aggregate on feeder cells or a medium containing bFGF. The method for producing embryonic stem cells is described, for example, in WO96/22362, WO02/101057, US5,843,780, US6,200,806, US6,280,718. The embryonic stem cells are available from a predetermined institution and also, commercially available. For example, human embryonic stem cells such as KhES-1, KhES-2 and KhES-3 are available from the Institute for Frontier Life and Medical Sciences, Kyoto University. Human embryonic stem cells such as Crx::Venus strain (derived from KhES-1) are available from RIKEN.

In the specification, the "induced pluripotent stem cells" refers to cells having pluripotency, which is induced by reprogramming somatic cells by a method known in the art.

The induced pluripotent stem cells were established in mouse cells by Yamanaka et al., in 2006 (Cell, 2006, 126 (4), pp. 663-676). The induced pluripotent stem cells were also established in human fibroblasts in 2007. The induced pluripotent stem cells have pluripotency and self-replicating ability similarly to embryonic stem cells (Cell, 2007, 131 (5), pp. 861-872; Science, 2007, 318 (5858), pp. 1917-1920; Nat. Biotechnol., 2008, 26 (1), pp.101-106).

The induced pluripotent stem cells more specifically refer to cells which are induced to be pluripotent by reprogramming somatic cells differentiated into, e.g., fibroblasts and peripheral blood mononuclear cells, by allowing any one of sets of a plurality of genes selected from a reprogramming gene group containing Oct3/4, Sox2, Klf4, Myc (c-Myc, N-Myc, L-Myc), Glis1, Nanog, Sall4, lin28 and Esrrb to express. Examples of a preferable set of reprogramming factors may include (1) Oct3/4, Sox2, Klf4, and Myc (c-Myc or L-Myc) and (2) Oct3/4, Sox2, Klf4, Lin28 and L-Myc (Stem Cells, 2013; 31: 458-466).

Other than producing induced pluripotent stem cells through direct reprogramming by gene expression, the pluripotent stem cells can be artificially induced from somatic cells, for example, by adding a chemical compound (Science, 2013, 341, pp. 651-654).

Alternatively, an induced pluripotent stem cell strain is available. For example, human induced pluripotent cell strains established by Kyoto University, such as 201B7 cell, 201B7-Ff cell, 253G1 cell, 253G4 cell, 1201C1 cell, 1205D1 cell, 1210B2 cell and 1231A3 cell, are available form Kyoto University and iPS Academia Japan, Inc. As the induced pluripotent stem cells, for example, Ff-I01 cell and Ff-I14 cell established by Kyoto University, are available from Kyoto University.

In the specification, the pluripotent stem cells are preferably embryonic stem cells or induced pluripotent stem cells, and more preferably induced pluripotent stem cells.

In the specification, the pluripotent stem cells are mammalian pluripotent stem cells, preferably pluripotent stem cells of a rodent (e.g., mouse, rat) or primates (e.g., human, monkey), more preferably human pluripotent stem cells and further preferably human induced pluripotent stem cells (iPS cells) or human embryonic stem cells (ES cells).

Pluripotent stem cells such as human iPS cells can be subjected to maintenance culture and expansion culture performed by methods known to those skilled in the art.

In the specification, the neural tissue is preferably derived from a human and preferably derived from pluripotent stem cells. A specific example of the neural tissue derived from pluripotent stem cells is neural tissue derived from embryonic stem cells or induced pluripotent stem cells. A more specific example of the neural tissue is neural tissue produced through differentiation induction from pluripotent stem cells such as embryonic stem cells or induced pluripotent stem cells (used as starting cells).

In the specification, the retinal tissue is preferably derived from pluripotent stem cells. A specific example of the retinal tissue derived from pluripotent stem cells is retinal tissue derived from embryonic stem cells or induced pluripotent stem cells. A more specific example of the retinal tissue is retinal tissue, starting from preferably pluripotent stem cells, more specifically embryonic stem cells or induced pluripotent stem cells, and produced through differentiation induction.

Neural tissue such as retinal tissue can be produced by using pluripotent stem cells in accordance with a method known to those skilled in the art. Examples of the method include those described in WO2011/055855, WO2011/028524, WO2013/077425, WO2015/025967, WO2016/063985 and WO2016/063986. Also, examples of the method include those described in, e.g., Non Patent Literature: Nature 472, pp51-56 (2011), Proc Natl Acad Sci U S A. 111 (23): 8518-8523 (2014), Nat Commun. 5: 4047 (2014), Nature Communications, 6, 6286 (2015), and Stem Cells. (2017); doi: 10.1002/stem. 2586.

Forebrain tissue and midbrain tissue can be produced from pluripotent stem cells in accordance with a method known to those skilled in the art. Examples of the method include Cell Stem Cell, 3,519-32 (2008), WO2009/148170 and WO2016/063985.

Multilayered neural tissue or retinal tissue can be produced not only by producing from pluripotent stem cells but also by dissociating cells such as neural precursor cells or neural retinal precursor cells derived from a living body and then reaggregating the dissociated cells.

The neural tissue or retinal tissue produced as mentioned above forms a cell aggregate. In an embodiment, the cell aggregate exhibits features: having (1) a round shape, (2) a smooth surface, (3) no collapse in shape and (4) being dense within the aggregate.

In the specification, the "cell aggregate" refers to mass formed of a plurality of cells mutually adhered. Examples of the cell aggregate include, but are not particularly limited to, Embryoid body, Sphere, Spheroid and Organoid. The cell aggregate includes proliferative cells, non-proliferating cells (cells stopped growing) or both of them.

In an embodiment, the "cell aggregate" has the aforementioned neuroepithelial structure. In an embodiment, the cell aggregate has one or more neuroepithelial structures.

In an embodiment, the retinal tissue produced by the above method has a structure in which one or (at least) a plurality of types of retinal cells are three-dimensionally arranged like a layer (vesicular lamellar morphology).

Whether retinal tissue has a vesicular lamellar morphology or not can be determined by those skilled in the art through observation of the retinal tissue with a microscope in the bright-field, more specifically, by confirming that the periphery of the aggregate of retinal tissue is bright and a vesicular or cup-like structure is present.

In the case of the neural tissue herein having a neuroepithelial structure, the neural tissue has a continuous epithelium structure in an embodiment. The continuous epithelium structure refers to a structure where the epithelium is continuously formed. The epithelium continuously formed means that 10 to 10000000 cells, preferably 30 to 10000000 cells, and further preferably 100 to 10000000 cells, are aligned, for example, in the tangent direction of the epithelium.

For example, in the continuous epithelium structure formed in retinal tissue, the retinal tissue has an apical surface intrinsic to the epithelium. The apical surface is formed almost in parallel to, e.g., at least photoreceptor layer (outer nuclear layer) among the layers forming a neural retinal layer and continuously on the surface of the retinal tissue. For example, in the case of a cell aggregate containing retinal tissue prepared from pluripotent stem cells, the apical surface is formed on the surface of the cell aggregate by regularly and continuously aligning, e.g., 10 or more, preferably 30 or more, more preferably 100 or more and further preferably 400 or more of photoreceptor cells or photoreceptor precursor cells in the tangent direction of the surface.

In the specification, the tangential direction of the epithelium refers to the direction along which individual cells are arranged, in other words, refers to the direction in parallel to the epithelium (or epithelial sheet) or the lateral direction.

In an embodiment, the retinal tissue produced by the aforementioned method has a rosette-like structure. In the specification, the "rosette-like structure" in retinal tissue refers to a structure where cells are radially or helically arranged so as to surround a central lumen. In retinal tissue in which a rosette-like structure is formed, the apical surface and photoreceptor cells or photoreceptor precursor cells are present along the central lumen and the apical surface is independently formed for each rosette-like structure.

In the specification, the "apical surface" refers to the surface (upper surface layer) rich in mucopolysaccharide (PAS staining-positive) in the epithelium tissue, and formed on the opposite side, i.e., basal membrane side, on which a basal side layer (basal membrane) rich in laminin and IV-type collagen, having a thickness of 50-100 nm and produced by epithelial cells, is present. In an embodiment, in the retinal tissue developed to the extent that photoreceptor cells or photoreceptor precursor cells are observed, the "apical surface" refers to a surface in contact with photoreceptor layer (outer nuclear layer) in which external limiting membrane is formed and photoreceptor cells and photoreceptor precursor cells are present. The apical surface can be identified by, e.g., immunostaining (known to those skilled in the art) using an antibody against an apical-surface marker (e.g., atypical-PKC (hereinafter referred to as "aPKC"), E-cadherin, N-cadherin).

Whether neural tissue has a continuous epithelium structure or not can be checked by determining continuity (i.e., non-dissociation form) of the apical surface of the neural tissue. Continuity of the apical surface can be determined by immunostaining of a marker of the apical surface, such as aPKC, E-cadherin and N-cadherin or by staining of a cell nucleus (e.g., DAPI staining, PI staining and Hoechst staining or staining of a marker protein (e.g., Rx, Chx10, Ki67 and Crx) localized in the cell nucleus).

The retinal tissue can be determined by immunostaining a marker on the apical surface (e.g., aPKC, E-cadherin, N-cadherin), a marker (e.g., Crx or recoverin) for photoreceptor cells or photoreceptor precursor cells present on the apical side; and analyzing positional relationship between the apical surface, photoreceptor layer and individual retinal layers in e.g., obtained images. Continuity of the retinal layers except the apical surface and photoreceptor layer (outer nuclear layer) can be determined by staining of cell nucleus, such as DAPI staining, PI staining and Hoechst staining or immunostaining of a marker protein (e.g., Rx, Chx10, Ki67, Crx) localized in the cell nucleus.

Whether neural tissue has a rosette-like structure or not can be determined by, e.g., fixing a cell aggregate with 4% paraformaldehyde, freezing and slicing to prepare tissue sections; immunostaining the sections with an antibody against an apical-surface marker such as aPKC, E-cadherin or N-cadherin, or staining with a dye such as DAPI, which specifically stains nuclei, in accordance with, e.g., an immunostaining method routinely carried out; and observing dysplasia (e.g., dissociated apical surface or invasion of the apical surface into a cell aggregate) of the rosette-like structure.

In the neural tissue produced by the aforementioned method, neural cells as mentioned above are present. The ratio of the predetermined types of neural cells varies depending on the differentiation stage.

The forebrain tissue produced by the aforementioned method is partly present as an aggregate having a lumen positive to Otx2.

The midbrain tissue produced by the aforementioned method is partly present as an aggregate having a lumen positive to Otx2.

In the retinal tissue produced by the aforementioned method, e.g., Chx10-positive neural retinal precursor cells and Crx-positive photoreceptor precursor cells are present. The ratio of these cells varies depending on the differentiation stage. In an embodiment, in the retinal tissue in a certain differentiation stage, Crx-positive photoreceptor precursor cells are present (like a layer) as a photoreceptor layer in the surface layer (apical surface side) of the retinal tissue. Inside the photoreceptor layer, Chx10-positive neural retinal precursor cells are present (like a layer) as a precursor cell layer (also referred to as neuroblastic layer).

In the specification, the "preservation solution" for use in preserving or transporting neural tissue has a potassium ion concentration of more than 0 mM and less than 115 mM. The preservation solution is not particularly limited as long as it is an aqueous solution suitable for survival of animal cells or animal tissues; however, the preservation solution preferably contains a buffering agent.

The buffering action refers to an action to keep hydrogen ion concentration at a constant level when a weak acid is mixed with its conjugate base or when a weak base is mixed with its conjugate acid. In other words, the buffering action refers to an action to keep pH of an aqueous solution within a constant range even if external factors in the aqueous solution changes. Examples of the possible change of external factors include addition of a small amount of acidic or basic substance in an aqueous solution, dilution of the solution, a change of CO₂ concentration in the atmosphere, production of metabolite from cells or tissues and a change in temperature.

The buffering agent refers to a substance responsible for keeping pH within a constant range by interacting other components in the aqueous solution, thereby bringing buffering action to the aqueous solution. More specifically, the preservation solution in the specification refers to an aqueous solution having buffering action to keep a pH within the range of 6.0 to 8.6. The pH of the preservation solution falls within the range of preferably 6.2 to 8.4, more preferably 6.7 to 7.9, more preferably 7.2 to 7.8 and further preferably 7.2 to 7.4.

In the specification, a buffering agent contained in a preservation solution is not particularly limited as long as it can keep the aqueous solution within the above pH range; and examples thereof include a combination of a weak acid and its conjugate base (for example, an alkali metal salt such as sodium or potassium, an alkaline earth metal salt such as calcium or magnesium, an ammonium salt) and a combination of a weak base and its conjugated acid (for example, an amine compound and a hydrochloride). One or more substances known to those skilled in the art can be used appropriately in combination. Examples of the buffer include, but are not limited to, a carbonate buffer (sodium hydrogen carbonate, sodium carbonate), a phosphate buffer (phosphoric acid, potassium phosphate, sodium phosphate, disodium hydrogen phosphate, dipotassium hydrogen phosphate, sodium dihydrogen phosphate, potassium dihydrogen phosphate, trisodium phosphate, tripotassium phosphate), good buffer (e.g., HEPES, MES, PIPES), tris, a citrate buffer (trisodium citrate), an acetate buffer (sodium acetate, potassium acetate), a borate buffer (sodium borate, sodium tetraborate), tartaric acid (sodium tartrate), and an amino acid buffer (histidine, taurine, aspartic acid). In an embodiment, one or a combination of buffering agents appropriately selected from a plurality of buffering agents may be contained in the preservation solution. Examples of a preferable buffering agent include sodium hydrogen carbonate, a phosphate buffer (sodium dihydrogen phosphate and disodium hydrogen phosphate) and HEPES. In an embodiment, as a buffering agent to be contained in the preservation solution, sodium hydrogen carbonate and one or more buffering agents appropriately selected from other buffering agents may be contained in combination.

In the specification, the aqueous solution refers to a solution basically containing water (H₂O) as a solvent. An aqueous liquid may be contained in a small amount to the extent that it does not affect survival of cells.

In the specification, the preservation solution for use in preservation of neural tissue may contain an inorganic salt other than a buffering agent.

Examples of the inorganic salt to be contained in the preservation solution include an inorganic salt, which is known as a component contained in the living body of mammals and a component known to be useful for survival of cells. Examples thereof include metal salts of nitric acid, sulfuric acid and hydrochloric acid. Note that, in the specification, the inorganic acid bringing buffering action to an aqueous solution is regarded as a buffering agent. The metal salt is not particularly limited as long as it forms a stable inorganic salt and it is useful for survival of cells. Examples of the metal salts include alkali metal (sodium, potassium) salts, alkaline earth metal (calcium, magnesium) salts and salts of e.g., copper, zinc and iron. Specific examples thereof include a potassium salt, a sodium salt, a calcium salt, a magnesium salt and an iron salt, and more specifically, potassium chloride, sodium chloride, calcium chloride, iron nitrate and magnesium chloride.

In the specification, the preservation solution may contain a potassium salt as a buffering agent or as an inorganic salt serving for another action. The potassium ion concentration contained in the preservation solution is not particularly limited as long as it is more than 0 mM or less than 115 mM concentration; preferably 60 mM, more preferably 30 mM and further preferably less than 10 mM. The potassium ion concentration is preferably 0 mM to 60 mM, more preferably 0 mM to 30 mM, further preferably 0.01 mM to 20 mM, further preferably 0.1 mM to 10 mM and further preferably 1 mM to 8 mM.

In the specification, the ratio of potassium ion concentration to sodium ion concentration in the preservation solution is preferably less than 0.8, further preferably 0.5, more preferably 0.4, 0.3, 0.2, 0.1, and further preferably less than 0.05.

In the specification, the "potassium ion concentration" refers to the concentration of inorganic ions in a preservation solution when all salts present in the preservation solution are dissociated into ions. The value does not depend on the state where a potassium salt is dissociated or not in the preservation solution. For example, if potassium phosphate (K₃PO₄) and potassium chloride (KC1) are both contained in a preservation solution and further assumed that potassium ion (K⁺) and phosphate ion (PO₄³⁻) are completely dissociated and potassium ion (K⁺) and chloride ion (Cl⁻) are completely dissociated, the concentration of the total potassium ions derived from both salts is defined as the concentration of potassium ions contained in the preservation solution.

In the specification, one example of the important properties that the preservation solution should satisfy can be osmotic pressure. An example of a factor influencing the osmotic pressure of the preservation solution is concentration of a buffering agent and other organic salts. The osmotic pressure of the preservation solution is preferably controlled to the extent that a subject to be preserved, i.e., neural tissue, does not swell or shrink during the preservation period. In an embodiment, the preservation solution refers to an aqueous solution having such an osmotic pressure that a preservation target, i.e., neural tissue, does not swell or shrink. The preservation solution is preferably an aqueous solution having buffering action so as to control pH within the range of 6.0 to 8.6 and an osmotic pressure to the extent that neural tissue does not swell or shrink.

The osmotic pressure to the extent that neural tissue does not swell or shrink is more specifically 200 to 500 mOsm/kg, for example, 200 to 400 mOsm/kg or 250 to 360 mOsm/kg. The osmotic pressure of the preservation solution is defined by the concentration of the whole substances including ions contained in the preservation solution. The osmotic pressure of the preservation solution can be appropriately controlled by changing the content of, e.g., ions, contained in the preservation solution or may be controlled by adding an osmoregulating chemical (for example, hydroxyethyl starch). The osmotic pressure can be measured, for example, by a commercially available osmotic pressure measuring device.

In the specification, the preservation solution may contain at least one additive appropriately selected from nutritional components such as amino acids, vitamins, sugar, nucleobases and pyruvates; antibiotic substances (for example, penicillin, streptomycin, gentamicin, amphotericin); steroids such as cholesterol; 2-mercaptoethanol, serum and serum replacement, other than a buffering agent and inorganic salts other than the buffering agent, as long as the cells contained in neural tissue can be survived and the neural tissue can be preserved; however, the additives are not limited to these. More specifically, components contained in culture medium and listed in Table 1 to Table 9, are mentioned as examples.

As the preservation solution, e.g., commercially available preservation solution and culture mediums for cells or biological tissues, satisfying the aforementioned conditions can be appropriately used. In the specification, the higher the ability of the preservation solution to maintain shape and properties of neural tissue without changing, the more preferable. In this sense, the lower the cell-proliferation or differentiation ability of the preservation solution, the more preferable. Whether cells proliferate in preservation solution or not can be checked by a method known to those skilled in the art, such as immunostaining of a cell proliferation marker, Ki67 or uptake rate of a nucleic acid analog (e.g., tritium labeled product, BrdU, CldU, IdU, EdU).

In an embodiment, the preservation solution can contain a glycosaminoglycan. An example of the glycosaminoglycan is a linear polysaccharide having a basic skeleton containing an amino sugar and uronic acid. In the specification, the glycosaminoglycan to be used in a preservation solution is not particularly limited as long as it can be dissolved in water and it does not adversely affect viability of neural tissue. In the specification, preferable examples of the glycosaminoglycan include chondroitin sulfate and hyaluronic acid. An advantage of the content of a glycosaminoglycan in a preservation solution is that the preservation solution becomes viscous and transportation vibration can be reduced.

The concentration of a glycosaminoglycan in the preservation solution, i.e., the concentration of total glycosaminoglycans is, for example, 10% (w/v) or less, 4% (w/v) or less, 3% (w/v) or less or 2.5% (w/v) or less. The concentration of a glycosaminoglycan is, for example, 0.1% (w/v) or more, 0.2% (w/v) or more, 0.5% (w/v) or more, or 0.7% (w/v) or more. In other words, the concentration of total glycosaminoglycans falls within the range of, for example, 0.1% (w/v) or more and 10% (w/v) or less, 0.2% (w/v) or more and 4% (w/v) or less, 0.5% (w/v) or more and 3% (w/v) or less, and 0.7% (w/v) or more and 2.5% (w/v) or less.

When chondroitin sulfate is contained as a glycosaminoglycan in the preservation solution, the concentration of chondroitin sulfate in terms of sodium chondroitin sulfate is, for example, 10% (w/v) or less, 4% (w/v) or less, 3% (w/v) or less or 2.5% (w/v) or less. The concentration of chondroitin sulfate in terms of sodium chondroitin sulfate is, for example, 0.1% (w/v) or more, 0.2% (w/v) or more, 0.4% (w/v) or more, or 0.5% (w/v) or more. The concentration of chondroitin sulfate in terms of sodium chondroitin sulfate falls within the range of, for example, 0.1% (w/v) or more and 10% (w/v) or less, 0.2% (w/v) or more and 4% (w/v) or less, 0.4% (w/v) or more and 3% (w/v) or less, and 0.5% (w/v) or more and 2.5% (w/v) or less.

As the glycosaminoglycan, a single glycosaminoglycan can be used; however, a plurality of types of glycosaminoglycans may be used in combination. For example, chondroitin sulfate and hyaluronic acid can be used in a weight ratio of 1:1 to 3:1 or 2:1 to 3:1.

In an embodiment, the preservation solution may have low activity for promoting metabolism of cells. The metabolic state of cells can be evaluated by measuring the amounts of metabolic pathway substances (for example, starting substance, intermediate, final metabolite). The amounts of substances on the metabolic pathway can be measured by liquid chromatography, gas chromatography, gel filtration chromatography, NMR or by an analytical instrument such as a mass spectrometer, a UV spectrometer or an IR spectrometer, or combination use of these analytical instruments.

In an embodiment, the preservation solution may contain a serum and or a serum substitute; however, the concentration thereof may be low. If the concentration of the serum and or serum substitute contained in the preservation solution is low, the effect of suppressing proliferation of cells or metabolism of cells can be expected.

As the preservation solution, a culture medium for use in maintenance-culture of cells or tissues can be used. A medium for culturing cells or tissues, preferably, an aqueous solution commercially available as medium for culturing animal cells or animal tissues can be used as the preservation solution of the present invention, as long as proliferation or differentiation of cells, by which the form of neural tissue substantially changes at a preservation temperature of the present invention, does not occur in the aqueous solution. Examples of the medium include mediums for use culturing animal cells such as BME medium, BGJb medium, CMRL 1066 medium, Glasgow MEM (GMEM) medium, Improved MEM Zinc Option medium, IMDM medium, Medium 199 medium, Eagle MEM medium, αMEM medium, DMEM medium, F-12 medium, DMEM/F12 medium, IMDM/F12 medium, Neurobasal medium, Ham's medium, RPMI 1640 medium and Fischer's medium and a mixture of these mediums. Preferable examples thereof include DMEM (Gibco), DMEM/F-12, Neurobasal medium (Thermo Fisher Scientific K.K.) and a mixture of these medium. These mediums can be used for maintenance culture of cells or tissues by appropriately adding, e.g., a serum, a serum substitute and an antibiotic substance. However, a serum, a serum substitute and/or an antibiotic substance are not essential components for the preservation solution. For the reason, when these mediums are used as the preservation solution of the present invention, these substances are preferably not added in order to reduce the number of items being quality controlled in the preservation solution. Even if these substances are added, the concentrations of these substances to be added should be low compared to those used in ordinary cell cultures. In an embodiment, as the preservation solution, e.g., D-PBS containing a buffering agent and an inorganic salt and not containing amino acids, vitamins or saccharides, can be used.

In an embodiment, the preservation solution may preferably contain a buffering agent and an inorganic salt as well as amino acids and/or vitamins. More preferable examples of the preservation solution include an aqueous solution containing a buffering agent, amino acids and vitamins. Specific examples thereof include HBSS (available from Thermo Fisher Scientific K.K.), DMEM (available from Thermo Fisher Scientific K.K.), DMEM-no-glucose (available from Thermo Fisher Scientific K.K.), Neurobasal medium (available from Thermo Fisher Scientific K.K.), corneal preservation solution having the composition shown in the following Table 1 (see, U.S. Patent Nos. 5,104,787 and 5,407,669) and Optisol (available from Bausch & Lomb Incorporated.). Optisol is more preferable.

**[Table 1]**

| Inorganic salts | (mg/L) | (mM) |
|---|---|---|
| Calcium chloride (CaCl₂) | 200 | 1.80 |
| Iron nitrate Fe(NO₃)₃ 9H₂O | 0.5 | 0.00124 |
| Potassium chloride (KCl) | 400 | 5.33 |
| Magnesium sulfate (MgSO₄ 7H₂O) | 200 | 0.813 |
| Sodium chloride (NaCl) | 6800 | 117 |
| Sodium hydrogen carbonate (NaHCO₃) | 2200 | 26.2 |
| Sodium dihydrogen phosphate (NaH₂PO₄ H₂O) | 140 | 1.01 |
| Other main components contained | | |
| Amino acids, vitamins, adenine sulfate, cholesterol, glucose, phenol red, sodium pyruvate, 2-mercaptoethanol, chondroitin sulfate, gentamicin, dextran, and the like. | | |

In an embodiment, the present invention provides a composition for transplantation containing a preservation solution and human neural tissue and controlled at a temperature of 12°C to 22°C, preferably 15°C to 20°C, i.e., 12°C to 22°C or 15°C to 20°C.

An example of the preservation solution herein is an aqueous solution having a potassium ion concentration of more than 0 mM and less than 115 mM and pH within the range of 6.0 to 8.6, which is controlled by a buffering agent. The aqueous solution may further contain amino acids and vitamins. An example of the aqueous solution is an aqueous solution for maintaining or culturing cells and tissues and having a potassium ion concentration of more than 0 mM and less than 115 mM and pH falling within the range of 6.0 to 8.6, which is controlled by a buffering agent. Specific examples thereof include Optisol, HBSS, DMEM, DMEM-no-glucose, Neurobasal medium and a corneal preservation solution having the composition shown in Table 1.

The aqueous solution preferably further contains a glycosaminoglycan such as chondroitin sulfate and/or hyaluronic acid. The concentration of a glycosaminoglycan herein is the same as defined above.

An example of the human neural tissue is a human neural tissue derived from pluripotent stem cells, for example, a human neural tissue that is a cell aggregate having a neuroepithelial structure.

Specific examples of the cell aggregate include a retinal tissue having a continuous epithelium structure on a cell aggregate surface, and forebrain/midbrain tissue having a continuous epithelium structure on the surface or inside a cell aggregate and/or a rosette structure. An example of the retinal tissue is the aforementioned retinal tissue such as neural retinal tissue having a continuous epithelium structure containing CRX and/or N-cadherin positive cells. An example of the forebrain/midbrain tissue is a forebrain/midbrain tissue having a continuous epithelium structure containing Otx2-positive cells and/or a rosette (lumen) structure.

The preservation solution for use in the preservation method or transport method of the present invention may be changed in composition in the middle of the preservation period as long as it can preserve neural tissue. The preservation solution may be exchanged with a (fresh) preservation solution having the same composition, in the middle of the preservation period. Examples of the ratio of exchange include, but are not limited to, 50%, 80% and 100%.

The container for use in the preservation method or transport method of the present invention is not particularly limited and can be appropriately determined by those skilled in the art. Examples of the container include a flask, a flask for tissue culture, a culture dish (dish), a petri dish (schale), a dish for tissue culture, a multi-dish, a microplate, a microwell plate, a micropore, a multi plate, a multi-well plate, a chamber slide, a tube, a tube with a screw cap, a vial, a tray and a culture bag. These containers are preferably non-cell-adhesive in order to successfully preserve neural tissue while keeping the neural tissue suspended therein. As the non-cell-adhesive container, e.g., a container having an untreated inner surface, to which a treatment for improving adhesion to cells (for example, coating with an extracellular matrix such as a basal membrane preparation, laminin, entactin, collagen or gelatin or a polymer compound such as polylysine or polyornithine; or a surface treatment with a positive charge) is not artificially applied, can be used. As the non-cell-adhesive container, a container having a surface, to which a treatment for reducing adhesion to cells (for example, treatment with a super hydrophilic MPC polymer or a treatment for reducing protein adsorption) is applied, can be used. Examples of the material for a container include, but are not limited to, polystyrene and polypropylene. The bottom of a container may be flat or uneven.

In an embodiment, the container is preferably aseptic. As a method for sterilizing a container, a method known to those skilled in the art can be used. Alternatively, a commercially available aseptic container may be used.

In an embodiment, as the container, a tube for preserving cells may be used. Examples of the tube for preserving cells include, but are not limited to, a cryotube, a conical tube, a centrifuge tube and a plastic tube.

In the preservation method or transport method of the present invention, neural tissue is preserved at a preservation temperature of 8°C or more or 12°C or more and 30°C or less or 22°C or less; and in other words, from 8°C to 30°C, preferably from 12°C to 22°C, more preferably about 17°C (from 15°C to 19°C or from 16°C to 18°C). To control the preservation temperature, a thermoregulator (described later) may be used.

In an embodiment, the preservation temperature may be the temperature of the preservation solution actually measured by, e.g., a thermometer, an actual measurement value of temperature within the thermoregulator, or a preset temperature of a thermoregulator. Temperature can be measured, for example, by a thermometer.

The thermoregulator is not particularly limited as long as it has a function to keep the temperature of the gas phase or liquid phase kept airtight within the aforementioned range. Examples of the apparatus that can be used herein include a CO₂ incubator for culturing cells, a refrigerator, a centrifuge, a constant-temperature water tank, a thermostatic chamber, a block incubator and heat insulation gel. The other conditions (e.g., oxygen concentration, carbon dioxide concentration) can be appropriately determined by those skilled in the art depending on, e.g., the tissue to be preserved.

Neural tissue is sometimes transported between facilities (e.g., from a neural tissue production facility to a neural tissue transplantation facility). It is preferable that neural tissue is suitably preserved during the transport period and the preservation method disclosed in the specification can be applied. The transport method, which is not particularly limited, may be any one of the means such as a vehicle, a ship and an aircraft. It is preferable that a transport means has a suitable thermoregulator or that a transport means can transport including a thermoregulator. It is also preferable that the thermoregulator has a structure preventing propagation of vibration by transport to the preservation solution loaded therein. Accordingly, a method for transporting neural tissue preserved in the preservation solution having a potassium ion concentration of more than 0 mM and less than 115 mM at a temperature of 8°C to 30°C, preferably 12°C to 22°C, and more preferably about 17°C (from 15°C to 19°C or from 16°C to 18°C) also falls within the scope of the present invention.

In the specification, preservation of neural tissue is initiated by placing neural tissue in a thermoregulator controlled to fall within the above temperature range and terminated by taking out the neural tissue out of the thermoregulator.

If the preservation solution of the present invention is used, neural tissue can be preserved for a period of not more than 14 days, not more than 5 days or not more than 3 days. The period, since time required for quality control or transport until the neural tissue is subjected to transplantation must be included, is usually 2 hours or more. In the specification, "for a period of not more than X days" refers to a period from the preservation initiation date, which is defined as Day 0, to Day X. In an embodiment, neural tissue may be preserved in accordance with the method of the present invention for less than one month although there is a possibility of quality deterioration of the neural tissue.

In the preservation method or transport method of the present invention, preservation of neural tissue is discriminated from a culture step. In the culture step of neural tissue (for example, maintenance culture or differentiation induction culture), e.g., metabolic activity of cells, proliferation, fate determination, differentiation, maturation and migration occur intermittently. In the culture step (for example, maintenance culture, differentiation induction culture), neuronal cells are cultured in the conditions suitable for survival and proliferation or differentiation induction of the neuronal cells. Examples of the conditions include a medium composition (containing nutrition components such as saccharides, amino acids, vitamins), temperature (usually 36 to 37°C), humidity (usually 95%), carbon dioxide concentration (usually 5 to 10%) and oxygen concentration (usually 2 to 60%).

The preservation temperature of the present invention is 30°C or less and differs from the optimum temperature in the culture step (for example, maintenance culture or differentiation induction culture of neural tissue). In the preservation according to the present invention, it is not essential to maintain and control nutrition components in a medium, humidity and carbon dioxide concentration.

In the specification, in the preservation of neural tissue, it is preferable that the states of neural tissue before and after preservation are substantially the same without substantial proliferation and differentiation of cells. For example, when neural tissue is used for transplantation, the quality of the produced neural tissue is sometimes tested. For the quality test, several hours and several days are required although the period varies depending on the items to be checked. The fact that the states of neural tissue before and after preservation are equivalent can be demonstrated by conducting quality tests that can be set by those skilled in the art. In an embodiment, the fact that the states of neural tissue before and after preservation are substantially equivalent can be demonstrated by comparing the effects of neural tissues transplanted to animal models.

When neural tissue is produced in a production facility such as the CPC cell culture center, it is necessary to transport the neural tissue to a medical institution at which a patient (recipient) desiring transplantation is hospitalized, however, if the institution is distant, several hours to several days are presumably required for transport.

It is important that neural tissue is kept substantially in the equivalent state during the period of conducting quality test or transporting it.

In an embodiment, the "preservation" of neural tissue is distinguished from "culture" accompanied with cell proliferation. During the maintenance culture or differentiation induction, proliferation or differentiation of cells is intermittently carried out. Accordingly, a medium composition (containing nutrition components such as saccharides, amino acids, vitamins) and external environments such as temperature (usually 37°C), humidity (usually 95%) and carbon dioxide concentration (usually 5%), suitable for survival and proliferation or differentiation induction of cells are required. In the specification, the "preservation" refers to keeping a cell aggregate without change in nature and without substantial proliferation and differentiation of cells; in other words, keeping a substantially equivalent state.

In an embodiment, the "preservation" of neural tissue is distinguished from "culture" accompanied with cell proliferation. When tissue is "cultured", the morphology of the tissue sometimes changes (for example, elongation, intrusion, inversion). Accordingly, a medium composition (containing nutrition components such as saccharides, amino acids, vitamins) and external environments such as temperature (usually 37°C), humidity (usually 95%) and carbon dioxide concentration (usually 5%), suitable for culture of cells are required.

In the preservation method of the present invention, the preservation temperature is 30°C or less, which differs from the optimum temperature of cell proliferation or differentiation.

In an embodiment, it is preferable that survival rate of neuronal cells contained in neural tissue before and after preservation by the method disclosed in the specification are the same. The phrase "survival rate of cells ... are the same" means that the ratio of survival rate of neuronal cells preserved relative to survival rate of neuronal cells before they are brought into contact with the preservation solution, does not substantially change; and more specifically, means that the survival rate of neuronal cells is 50% or more, 60% or more, 70% or more, 80% or more, 90% or more or 95% or more.

Herein, if the survival rate of neuronal cells contained in the neural tissue to be used for transplantation before bringing them into contact with the preservation solution of the present invention is, for example, 95% or more,
the survival rate of neuronal cells in the tissue preserved by the method of the present invention, more specifically, preserved in the preservation solution for several hours to 14 days, is maintained at 80% or more, 90% or more or 95% or more, although the survival rate varies depending the preservation period and preservation conditions.

In an embodiment, when a cell aggregate containing retinal tissue is preserved by the method of the present invention, the numbers of CRX-positive cells in the cell aggregate before and after the preservation are the same. The phrase "the numbers of CRX-positive cells ... are the same" means that the ratio of the number of CRX-positive cells, which are preserved in the preservation solution for several hours to 14 days, relative to the number of CRX-positive cells before they are brought into contact with the preservation solution of the present invention, is maintained at 70% or more, 80% or more or 95% or more. although the ratio varies depending on the preservation period and preservation conditions.

In an embodiment, when a cell aggregate containing retinal tissue is preserved by the method of the present invention, the numbers of cell aggregates having an Otx2-positive rosette structure (lumen) before and after preservation are the same. The phrase herein "the numbers of cell aggregates having an Otx2-positive rosette structure ... are the same" means that, the ratio of the number of aggregates having an Otx2-positive lumen, which are preserved in the preservation solution for several hours to 14 days, relative to the number of aggregates having an Otx2-positive rosette structure, before they are brought into contact with the preservation solution of the present invention, is maintained at 50% or more, 80% or more or 90% or more, although the ratio varies depending on the preservation period and preservation conditions.

In an embodiment, if preservation is made by the method of the present invention, the layer structure or three-dimensional cell arrangement intrinsic to an aggregate of neural tissue can be maintained substantially the same before and after preservation. For example, in the case of retinal tissue containing a neural retinal layer, a more specific example thereof is cell arrangement where continuous epithelium structure, vesicular lamellar morphology and Crx-positive cells are present like a layer on the surface layer and Chx10-positive cells are present like a layer inside the above layer. The phrase "the layer structure or three-dimensional cell arrangement can be maintained substantially the same" means that the ratio of the number of aggregates having the layer structure or three-dimensional cell arrangement and preserved in the preservation solution for several hours to 14 days, relative to the number of aggregates having the layer structure or three-dimensional cell arrangement presenting a specific property before they are brought into contact with the preservation solution of the present invention, is 80% or more, preferably 90% or more and more preferably 95% or more. In another embodiment, the phrase "the layer structure or three-dimensional cell arrangement presenting a specific property is maintained the same" means that the ratio of the peripheral portion of an aggregate having the layer structure or three-dimensional cell arrangement compared to the ratio before they are brought into contact with the preservation solution of the present invention, is maintained at 70% or more, 80% or more or 95% or more, although the ratio varies depending on the preservation period and preservation conditions.

The neural tissue preserved by the preservation method can be immediately subjected to desired use, more specifically quality test and transplantation surgery to a patient (recipient). After a preservation period, neural tissue can be cultured for recovery in a medium for a short term (for example, 3 hours to 7 days) or long term (8 days to a few months). The recovery culture may be carried out by a method known to those skilled in the art, for example, in a CO₂ incubator set at 37°C. In an embodiment, the recovery culture can be carried out in exactly the same conditions as used in conventional differentiation culture or maturation culture.

The neural tissue such as forebrain tissue, midbrain tissue or retinal tissue, preserved by the preservation or the transport method of the present invention, can be used, for example, transplantation into a living body (recipient).

In an embodiment, when the neural tissue preserved such as forebrain tissue, midbrain tissue or retinal tissue, is transplanted into a living body, the neural tissue taken out from the preservation solution may be directly transplanted. In an embodiment, the neural tissue preserved may be washed with an agent usually used in a medicine containing a tissue or cells, such as a preservative, a stabilizer, a reducing agent and a tonicity agent, and transplanted by using these as a carrier.

The neural tissue (e.g., retinal tissue, forebrain tissue, midbrain tissue) preserved by the preservation method or the transport method of the present invention, is useful for medical transplantation for a disease caused by damage of the neural tissue. Then, the present invention provides a therapeutic product containing neural tissue preserved by the preservation method of the present invention, for a disease caused by damage of the neural tissue. As a therapeutic product for a disease caused by damage of the neural tissue or in order to make up for a damaged site corresponding to a damaged neural tissue in damage state of neural tissue, neural tissue preserved by the preservation method of the present invention can be used. By transplanting neural tissue preserved by the preservation method of the present invention to make up for a damaged site or damaged neural tissue itself in a patient having a disease requiring transplantation, a disease caused by damage of neural tissue or damage state of neural tissue, it is possible to treat a disease caused by a damage of neural tissue or damage state of neural tissue. Examples of the disease caused by a damage of neural tissue include neurodegenerative diseases (for example, cerebral ischemic injury, cerebral infarction, Parkinson's disease, spinal cord injury, cerebrovascular disorder, brain/spinal traumatic disorder (e.g., cerebral infarction, head injury/brain contusion (traumatic head injury; TBI), spinal cord injury multisystem atrophy), typical neurodegenerative diseases (amyotrophic lateral sclerosis (ALS), Parkinson's disease (PD), Parkinson's syndrome, Alzheimer-type dementia, progressive supranuclear palsy (PSP), Huntington's disease, multiple system atrophy (MSA), spinocerebellar degeneration (SCD)), demyelinating disease, neuromuscular disease (multiple sclerosis (MS), acute disseminated encephalomyelitis (ADEM), inflammatory diffuse sclerosis (Schilder disease), subacute sclerosis panencephalitis, progressive multifocal leukoencephalopathy, hypoxic encephalopathy, central pontine myelinolysis, Binswanger disease, Guillain-Barre syndrome, Fisher syndrome, chronic inflammatory demyelinating polyradiculoneuropathy, syringomyelia, spinocerebellar degeneration, striato-nigral degeneration (SND), olivopontocerebellar atrophy (OPCA), Shy-Drager syndrome, ophthalmological diseases (macular degeneration, age-related macular degeneration, retinitis pigmentosa, cataract, glaucoma, corneal disease, retinopathy), intractable epilepsy, progressive supranuclear palsy, syringomyelia, spinal muscular atrophy (SMA), Spinal and Bulbar Muscular Atrophy (SBMA), primary lateral sclerosis (PLS), progressive supranuclear palsy (PSP), corticobasal degeneration (CBD), Huntington's disease (HD), chorea-acanthocytosis, syringomyelia, frontotemporal lobar degeneration, Charcot-Marie-Tooth disease, dystonia, Pantothenate kinase-associated neurodegeneration, familial dementia, Parkinson's syndrome, senile agnosia, spastic paraplegia, dementia with Lewy bodies, hypopituitarism and malfunction caused by surgical resection of a brain tumor. Examples of a disease based on a damage in cerebrum tissue or cerebral-related cell include neurodegenerative diseases (for example, cerebral ischemic insult, cerebral infarction, motor neuron disease, ALS, Alzheimer's disease, polyglutamine disease and corticobasal degeneration). Examples of a disease based on a damage in retinal tissue or retina related cells include retinal degeneration, retinitis pigmentosa, age-related macular degeneration, organic mercury poisoning, chloroquine retinopathy, glaucoma, diabetic retinopathy and neonatal retinopathy. Examples of the damage state of neural tissue include the state of a patient after neural tissue excision, the state of a patient who received X-ray irradiation to a tumor within neural tissue and trauma.

### Examples

Now, the present invention will be more specifically described by way of Examples; however, the present invention is not limited by these.

### Example 1: Preservation of cell aggregate containing retinal tissue prepared from human iPS cells in various preservation conditions (screening for preservation temperature and preservation solution)

Human iPS cells (1231A3 strain, obtained from Kyoto University) were subjected to feeder-free culture performed in accordance with the method described in Scientific Reports, 4, 3594 (2014). As the feeder-free medium, StemFit medium (AK03N, manufactured by AJINOMOTO CO., INC.) was used. As a feeder-free scaffold, Laminin 511-E8 (manufactured by Nippi. Inc.) was used.

Operation of maintenance culture was as follows: First, human iPS cells (1231A3 strain) reached sub-confluency were washed with PBS and separated into single cells by use of TrypLE Select (manufactured by Life Technologies). Then, the separated human iPS single cells were seeded in plastic culture dishes coated with Laminin 511-E8 and cultured in feeder-free StemFit medium in the presence of Y27632 (ROCK inhibitor, 10 µM). When 6-well plates (for cell culture, culture area: 9.4 cm², manufactured by AGC TECHNO GLASS., LTD) were used as the plastic culture dishes, the number of separated human iPS single cells to be seeded was specified as 1.0×10⁴. One day after seeding, the medium was exchanged with StemFit medium not containing Y27632. Thereafter, the medium was exchanged with Y27632-free StemFit once every 1 to 2 days. Thereafter the cells were cultured until the cells reached sub-confluency (state where about 60% of the culture area is covered by cells) on Day 6.

Operation of differentiation induction was carried out as follows: Human iPS cells (1231A3 strain) were cultured in feeder-free StemFit medium until the day before the cells reached sub-confluency (state where about 50% of the culture area is covered by cells). The human iPS cells the day before the sub-confluency were subjected to feeder-free culture for one day (preconditioning treatment) in the presence of SB431542 (5 µM) and SAG (300 nM).

The preconditioned human iPS cells were treated with TrypLE Select (manufactured by Life Technologies) and (further) separated into single cells by pipetting. Thereafter, the separated human iPS single cells were suspended in 100 µl of a serum-free medium such that the density of cells per well of a non-cell adhesive 96-well culture plate (PrimeSurface, 96 V-bottom plate, manufactured by Sumitomo Bakelite Co., Ltd.) was 1.2 × 10⁴ cells, and subjected to suspension culture in the conditions of 37°C and 5% CO₂. The serum-free medium (gfCDM + KSR) used herein is a serum-free medium prepared by adding 10% KSR and 450 µM 1-monothioglycerol and IX Chemically defined lipid concentrate to a mixture of culture fluids containing F-12 medium and IMDM medium in a ratio of 1:1. At the initiation time of the suspension culture (Day 0 from initiation of the suspension culture), IWR-le (final concentration 3 µM), Y27632 (final concentration 20 µM) and SAG (final concentration 30 nM) were added to the serum-free medium. Day 3 from initiation of the suspension culture, 50 µl of a fresh serum-free medium (the same one as mentioned above), which did not contain Y27632 or SAG and contained IWR-le and human recombinant BMP4 (manufactured by R&D), was added such that the final concentration of exogenous human recombinant BMP4 became 1.5 nM (55 ng/ml), and that the final concentration of IWR-le became 3 µM.

Three days later (that is, Day 6 from initiation of the suspension culture), a half of the medium was exchanged with the serum free medium, which did not contain Y27632 or human recombinant BMP4 and contained IWR-1e. The above half of the medium was exchanged with serum-free medium. Four days later (Day 10 from initiation of the suspension culture), 80% of the medium was exchanged with the serum-free medium, which did not contain IWR-1e, Y27632 or human recombinant BMP4. This medium exchange operation was repeated twice, such that the concentration of exogenous IWR-le became 3% or less compared to that before medium exchange. Thereafter, a half of the medium was exchanged with serum-free medium, which did not contain IWR-1e, Y27632 or human recombinant BMP4, once every 2 to 4 days. The operation for exchanging a half volume of the medium was as follows. A half volume, i.e., 75 µl, of the medium in the incubator was discarded, 75 µl of a fresh serum-free medium (the same one as mentioned above) was added to control the total medium volume to be 150 µl.

The cell aggregate obtained on Day 17 from initiation of the suspension culture was cultured in a serum free medium (prepared by adding 1% N2 supplement to DMEM/F12 medium) containing Wnt signal transducing pathway active substance, CHIR99021 (3 µM) and FGF signal transducing pathway inhibitor SU5402 (5 µM), for 3 days, i.e., up to Day 20 from initiation of the suspension culture.

Further, as a serum medium, which did not contain a Wnt signal transducing pathway active substance or an FGF signal transducing pathway inhibitor, a medium (hereinafter referred to as "Retina medium") was prepared by adding 10% fetal bovine serum, 1% N2 supplement, 0.5 µM retinoic acid and 100 µM taurine to DMEM/F12 medium. The cell aggregate on Day 20 from initiation of the suspension culture was subjected to suspension culture using Retina medium for 65 days, i.e., up to Day 85 from initiation of the suspension culture. During the period from Day 20 to Day 85 from initiation of the suspension culture, about a half volume of Retina medium was exchanged with fresh one once every 2 to 4 days.

The cell aggregate obtained on Day 8 from initiation of the suspension culture was subjected to observation by an inverted microscope (ECLIPSE Ti, manufactured by Nikon Corporation) as a bright-field image (phase contrast image). As a result, it was confirmed that the cell aggregate contains a three-dimensional neural tissue, which partly contains a continuous epithelium structure.

The following three types of preservation solutions were studied (see, Table 2 to 4).
- Retina medium: medium obtained by adding 10% fetal bovine serum, 1% N2 supplement, 0.5 µM retinoic acid, and 100 µM taurine into DMEM/F12 medium
- Hank's balanced salt solution (manufactured by Gibco, hereinafter referred to as "HBSS")
- Optisol-GS (registered trademark) (manufactured by Bausch & Lomb Incorporated., hereinafter referred to as "Optisol")

### [Table 2]

**Table 2 Components of DMEM/F12 medium**

| Inorganic salts (concentration) | (mg/L) | (mM) |
|---|---|---|
| Calcium chloride (CaCl₂) (non-hydrate) | 117 | 1.05 |
| Copper sulfate (CuSO₄-5H₂O) | 0.0013 | 0.0000052 |
| Iron nitrate (Fe(NO₃)₃ 9H₂O) | 0.05 | 0.000124 |
| Iron sulfate (FeSO₄-7H₂O) | 0.417 | 0.0015 |
| Magnesium chloride (MgCl₂) (non-hydrate) | 28.6 | 0.301 |
| Magnesium sulfate (MgSO₄) (non-hydrate) | 48.8 | 0.407 |
| Potassium chloride (KCl) | 311 | 4.16 |
| Sodium hydrogen carbonate (NaHCO₃) | 2438 | 29.0 |
| Sodium chloride (NaCl) | 7000 | 121 |
| Disodium hydrogen phosphate (Na₂HPO₄) (non-hydrate) | 71.02 | 0.500 |
| Sodium dihydrogen phosphate (NaH₂PO₄-H₂O) | 62.5 | 0.453 |
| Zinc sulfate (ZnSO₄-7H₂O) | 0.432 | 0.00150 |
| The other components contained | | |
| Amino acids, vitamins, glucose, hypoxanthine sodium, linolenic acid, lipoic acid, phenol red, putrescine dihydrochloride, sodium pyruvate, thymidine | | |

(Excerpt from Thermo Fisher Scientific K.K. web page: https://www.thermofisher.com/jp/ja/home/technical-resources/media- fo rmulation.58.html)

### [Table 3]

**Table 3 Components of HBSS**

| Inorganic salts (concentration) | (mg/L) | (mM) |
|---|---|---|
| Potassium chloride (KCl) | 400 | 5.33 |
| Potassium dihydrogen phosphate (KH₂PO₄) | 60 | 0.441 |
| Sodium hydrogen carbonate (NaHCO₃) | 350 | 4.17 |
| Sodium chloride (NaCl) | 8000 | 138 |
| Disodium hydrogen phosphate (Na₂HPO₄) (non-hydrate) | 48 | 0.338 |
| The other component contained | | |
| Glucose | | |

(Excerpt from Thermo Fisher Scientific K.K. web page: https://www.thermofisher.com/jp/ja/home/technical-resources/media- fo rmulation.1 56.html)

### [Table 4]

**Table 4 Components of Optisol**

| |
|---|
| Inorganic salts |
| Calcium chloride (CaCl₂) (non-hydrate) |
| Iron Nitrate Fe(NO₃)₃ 9H₂O |
| Potassium chloride (KCl) |
| Magnesium sulfate (MgSO₄) |
| Sodium chloride (NaCl) |
| Sodium hydrogen carbonate (NaHCO₃) |
| Sodium dihydrogen phosphate (NaH₂PO₄ H₂O) |
| Other main components contained |
| Amino acids, vitamins, adenine sulfate, cholesterol, glucose, phenol red, sodium pyruvate, 2-mercaptoethanol, chondroitin sulfate, gentamicin, streptomycin, dextran, and the like |

(Excerpt form safety information sheet Optisol GS manufactured by Bausch & Lomb Incorporated. in American Journal of Ophthalmology, 114, 345-356, (1992))

The following three preservation temperatures were studied.
- 37°C (incubator, 5% CO₂ condition, manufactured by Panasonic Corporation)
- 17°C (incubator, 5% CO₂ condition, manufactured by ASTEC Co., Ltd.)
- 4°C (refrigerator, sealed condition, manufactured by Panasonic Corporation)

All preservation containers studied were 60-mm low adhesive plates (manufactured by Sumitomo Bakelite Co., Ltd.).

The following A to I preservation conditions were specifically studied.
- Preservation condition A (ordinary culture condition): the preservation solution is Retina medium, the preservation temperature is 37°C, the preservation container is a low adhesive plate (5% CO₂)
- Preservation condition B: the preservation solution is Retina medium, the preservation temperature is 17°C, the preservation container is a low adhesive plate (5% CO₂)
- Preservation condition C: the preservation solution is Retina medium, the preservation temperature is 4°C, the preservation container is a low adhesive plate (sealed)
- Preservation condition D: the preservation solution is HBSS, the preservation temperature is 37°C, the preservation container is a low adhesive plate (5% CO₂)
- Preservation condition E: the preservation solution is HBSS, the preservation temperature is 17°C, the preservation container is a low adhesive plate (5% CO₂)
- Preservation condition F: the preservation solution is HBSS, the preservation temperature is 4°C, the preservation container is a low adhesive plate (sealed)
- Preservation condition G: the preservation solution is Optisol, the preservation temperature is 37°C, the preservation container is a low adhesive plate (5% CO₂)
- Preservation condition H: the preservation solution is Optisol, the preservation temperature is 17°C, the preservation container is a low adhesive plate (5% CO₂)
- Preservation condition I: the preservation solution is Optisol, the preservation temperature is 4°C, the preservation container is a low adhesive plate (sealed)

The cell aggregates on Day 85 from initiation of the suspension culture were preserved in preservation conditions A to I mentioned above for 48 hours. The cell aggregates (Day 87 from initiation of the suspension culture) were observed immediately after completion of preservation by an inverted microscope (ECLIPSE Ti, manufactured by Nikon Corporation) as a bright field image (phase contrast image).

As a result, it was found that a three-dimensional tissue form was maintained in almost all of the cell aggregates observed immediately after completion of preservation in preservation conditions A, B, E, F, G and H. In preservation conditions D and I, some of the cell aggregates maintained three-dimensional tissue form; however, a large number of cell aggregates had collapsed form. In preservation condition C, three-dimensional tissue form significantly collapsed in almost all of the cell aggregates.

Next, in order to check whether cells contained in the cell aggregate preserved can be normally cultured and whether differentiated cells cause abnormalities such as cell death, recovery culture was carried out. More specifically, cell aggregates preserved in preservation conditions A to I for 48 hours were subjected to suspension culture (recovery culture) performed in Retina medium using a low adhesive plate in an incubator in the conditions of 37°C and 5% CO₂.

The resultant cell aggregates obtained after recovery culture were observed by an inverted microscope (ECLIPSE Ti, manufactured by Nikon Corporation) as a bright field image (phase contrast image) (Figure 1).

As a result, neural tissue having a continuous epithelium structure having a layer-like structure was observed in almost all of the cell aggregates preserved in preservation conditions A, B, E, F, G and H and subjected to recovery culture. In the cases of preservation conditions D and I, a neural tissue having a layer-like continuous epithelium structure was observed; however, the form of cell aggregate (three-dimensional retina) collapsed in about 70% of the cell aggregates, as shown in Figure 1. The cell aggregates on average showed forms indicated by D and I. In contrast, in the case of preservation condition C, as shown in Figure 1, C, it was found that form of three-dimensional tissue tends to significantly collapse in almost all of the cell aggregates.

Each of the cell aggregates obtained after recovery culture was fixed with 4% paraformaldehyde, frozen and sectioned. The frozen sections obtained were subjected to immunostaining to stain a retinal tissue marker, Chx10 (anti-ChxlO antibody, Exalpha Biologicals, sheep) and a photoreceptor precursor cell marker Crx (anti-Crx antibody, Takara Bio Inc., rabbit). The nuclei of the cells were stained with DAPI. These sections stained were observed by a fluorescence microscope (manufactured by KEYENCE CORPORATION.) to obtain immunostained images (Figure 2 and Figure 3).

The immunostained images were morphologically analyzed. More specifically, whether neural retinal tissue having Crx-positive photoreceptor precursor cells are present like a layer on the surface layer and Chx10-positive neural retinal precursor cells are present inside the layer of the Crx-positive photoreceptor precursor cells, was checked. Further, whether the neural retinal tissue forms a continuous epithelium structure (hereinafter referred to as the "continuous epithelium structure of neural retinal tissue"), having a continuous length of 100 µm or more in the tangent direction of the periphery of the aggregate, was checked.

As a result, in cell aggregates preserved in preservation conditions A, B, E, F, G and H and subjected to recovery culture, it was found that the continuous epithelium structure of neural retinal tissue is present in almost all of the cell aggregates (Figure 2 and Figure 3). In preservation conditions D and I, the continuous epithelium structure of neural retinal tissue was observed in some of the cell aggregates; however, the three-dimensional form of the retina collapsed in about 70% of the cell aggregates, as shown in the figures. In preservation condition C, continuous epithelium structure of neural retinal tissue was rarely detected in almost all of the cell aggregates.

From these results, it was found that a cell aggregate can be preserved for 48 hours while maintaining the continuous epithelium structure of neural retinal tissue in preservation conditions B, E, F, G and H, compared to preservation condition A, which is the same condition in ordinary culture. It was also found that the continuous epithelium structure of neural retinal tissue can be partially maintained even in preservation conditions D and I.

In preservation conditions B, E and H, more specifically, in the condition of a preservation temperature of 17°C, it was found that stable preservation can be made in various types of preservation solutions.

### Example 2: Preservation of cell aggregates containing retinal tissue prepared from human ES cells in various preservation conditions (screening for preservation temperature and preservation solution)

Crx:: Venus knock-in human ES cells (derived from KhES-1; Nakano, T. et al. Cells Stem Cells 2012, 10 (6), 771-785; obtained from Kyoto University, established in RIKEN CENTER FOR DEVELOPMENTAL BIOLOGY and put in use) were cultured in StemFit medium in accordance with the method described in Example 1 in the feeder-free conditions until the day before the cells reached sub-confluency. Human ES cells of the day before the cells reached sub-confluency were cultured in feeder-free conditions in the presence of SB431542 (5 µM) and SAG (300 nM) for one day (preconditioning treatment).

The human ES cells preconditioned were treated with a cell separating agent, TrypLE Select (manufactured by Life Technologies) and separated into single cells by pipetting. Thereafter, the separated human ES single cells were suspended in 100 µl of a serum-free medium such that cells per well of a non-cell adhesive 96-well culture plate (PrimeSurface, 96 V-bottom plate, manufactured by Sumitomo Bakelite Co., Ltd.) was 1.2×10⁴ cells and subjected to suspension culture in the conditions of 37°C and 5% CO₂ (Day 0 from initiation of the suspension culture).

The serum-free medium (gfCDM+KSR) used herein is a serum-free medium prepared by adding 10% KSR and 450 µM 1-monothioglycerol and IX Chemically defined lipid concentrate to a mixture of culture fluids containing F-12 medium and IMDM medium in a ratio of 1:1.

At the initiation of the suspension culture (Day 0 from initiation of the suspension culture), Y27632 (final concentration 20 µM) and SAG (30 nM) were added in the serum-free medium. Day 3 from initiation of the suspension culture, 50 µL of a medium not containing Y27632 or SAG and containing human recombinant BMP4 (trade name: Recombinant Human BMP-4, manufactured by R&D) was added so as to obtain the final concentration of human recombinant BMP4 of 1.5 nM (55 ng/ml).

On Day 6 from initiation of the suspension culture of the cells prepared as described above, 60 µL of the medium was removed, 90 µL of fresh medium was added. On Day 9, 12, and 15 from initiation of the suspension culture, 85 µL of the medium was removed, 90 µL of the medium was newly added. The cells were cultured up to Day 18 from initiation of the suspension culture.

The cell aggregate on Day 18 from initiation of the suspension culture was cultured in serum-free medium (DMEM/F12 medium with 1% N2 supplement) containing CHIR99021 (3 µM) and SU5402 (5 µM) for 3 days, i.e., up to Day 21 from initiation of the suspension culture.

The resultant cell aggregate on Day 21 from initiation of the suspension culture was cultured in each of the serum mediums shown in the following [1], [2] and [3] in the condition of 5% CO₂ up to Day 80 from initiation of the suspension culture.
[1] Day 21 to day 40 from initiation of the suspension culture: DMEM/F12 medium containing 10% fetal bovine serum, 1% N2 supplement and 100 µM taurine (hereinafter referred to as "medium A").
[2] Day 40 to day 60 from initiation of the suspension culture: Mixture of culture fluids containing medium A and a medium, which was Neurobasal medium (see, Table 5) containing 10% fetal bovine serum, 2% B27 supplement, 200 mM glutamine and 100 µM taurine (hereinafter referred to as "medium B") in a ratio of 1:3.
[3] On and after Day 60 from initiation of the suspension culture: medium B.

The resultant cell aggregates on Day 80 from initiation of the suspension culture were observed by an inverted microscope (EVOS, manufactured by Thermo Fisher Scientific K.K.). As a result, it was found that a continuous epithelium structure of neural tissue was contained. Using the cell aggregates on Day 80 from initiation of the suspension culture, preservation conditions were studied.

The following 4 types of preservation solutions were studied.
- medium B
- HBSS (described in Example 1)
- Optisol (described in Example 1)
- University of Wisconsin solution (trade name: Viaspan, sold by Astellas Pharma Inc., hereinafter referred to as "UW solution"; see Table 6)

### [Table 5]

**Table 5 Components of Neurobasal medium**

| Inorganic salts (concentration) | (mg/L) | (mM) |
|---|---|---|
| Calcium chloride (CaCl₂) (non-hydrate) | 200 | 1.80 |
| Iron nitrate Fe(NO₃)₃ 9H₂O | 0.1 | 0.000248 |
| Magnesium chloride (MgCl₂) (non-hydrate) | 77.3 | 0.814 |
| Potassium chloride (KCl) | 400 | 5.33 |
| Sodium hydrogen carbonate (NaHCO₃) | 2200 | 26.2 |
| Sodium chloride (NaCl) | 3000 | 51.7 |
| Sodium dihydrogen phosphate (NaH₂PO₄ H₂O) | 125 | 0.906 |
| Zinc sulfate (ZnSO₄-7H₂O) | 0.194 | 0.000674 |
| The other components contained | | |
| Amino acids, vitamins, glucose, HEPES, phenol red, sodium pyruvate | | |

(Excerpt from Thermo Fisher Scientific K.K. web page: https://www.thermofisher.com/jp/ja/home/technical-resources/media- fo rmulation.251.html)

### [Table 6]

**Table 6 Components of UW solution**

| Inorganic ions (concentration) | (mM) |
|---|---|
| Sodium (Na) | 30 |
| Potassium (K) | 120 |
| Magnesium (Mg) | 5 |
| Sulfate ion | 5 |
| Phosphate ion | 25 |
| Lactobionate ion | 100 |
| The other components contained | |
| Raffinose, hydroxylethyl starch, glutathione | |

(Excerpt from the paper: World J Transplant. 2015 Dec 24; 5 (4): 154-164.)

The following three preservation temperatures were studied.
- 37°C (incubator, 5% CO₂ condition, manufactured by Panasonic Corporation)
- 17°C (incubator, 5% CO₂ condition, manufactured by ASTEC Co., Ltd.)
- 4°C (refrigerator, sealed condition, manufactured by Panasonic Corporation)

All preservation containers studied were 60-mm low adhesive plates (manufactured by Sumitomo Bakelite Co., Ltd.).

The following A to H preservation conditions were specifically studied.
- Preservation condition A (preservation condition in ordinary culture): the preservation solution is medium B, the preservation temperature is 37°C, the preservation container is a low adhesive plate (5% CO₂)
- Preservation condition B: the preservation solution is medium B, the preservation temperature is 17°C, the preservation container is a low adhesive plate (5% CO₂)
- Preservation condition C: the preservation solution is HBSS, the preservation temperature is 17°C, the preservation container is a low adhesive plate (5% CO₂)
- Preservation condition D: the preservation solution is HBSS, the preservation temperature is 4°C, the preservation container is a low adhesive plate (sealed)
- Preservation condition E: the preservation solution is Optisol, the preservation temperature is 17°C, the preservation container is a low adhesive plate (5% CO₂)
- Preservation condition F: the preservation solution is Optisol, the preservation temperature is 4°C, the preservation container is a low adhesive plate (sealed)
- Preservation condition G: the preservation solution is UW solution, the preservation temperature is 17°C, the preservation container is a low adhesive plate (5% CO₂)
- Preservation condition H: the preservation solution is UW solution, the preservation temperature is 4°C, the preservation container is a low adhesive plate (sealed)

The cell aggregates on Day 80 from initiation of the suspension culture were preserved in the above preservation conditions A to H for 48 hours. The cell aggregates were observed immediately after completion of preservation by an inverted microscope (ECLIPSE Ti, manufactured by Nikon Corporation) as a bright field image (phase contrast image).

As a result, three-dimensional tissue form was maintained in almost all of the cell aggregates obtained immediately after completion of preservation condition A (preservation condition in ordinary culture) and preservation conditions B, C and E. In preservation conditions D and F, some of the cell aggregates maintained three-dimensional tissue form; however, a large number of cell aggregates had collapsed form. In preservation conditions G and H using UW solution, three-dimensional tissue form significantly collapsed in almost all of the cell aggregates.

Next, cell aggregates preserved in conditions A to H were subjected to suspension culture performed by using medium B and a low adhesive plate in an incubator in the conditions of 37°C and 5% CO₂ (recovery culture).

The cell aggregates obtained after the recovery culture were observed by an inverted microscope (ECLIPSE Ti, manufactured by Nikon Corporation) as a bright field image (phase contrast image) (Figure 4).

As a result, in cell aggregates preserved in preservation condition A (preservation condition in ordinary culture) and preservation conditions B, C and E and subjected to the recovery culture, neural tissue having a layer-like continuous epithelium structure was highly frequently observed. In the cases of preservation conditions D and F, neural tissue having a layer-like continuous epithelium structure was found in some of the cell aggregates. In contrast, in preservation conditions G and H using UW solution, it was found that three-dimensional tissue form tends to significantly collapse in almost all of the cell aggregates.

Each of the cell aggregates obtained after recovery culture was fixed with 4% paraformaldehyde, frozen and sectioned. The frozen sections obtained were subjected to immunostaining to stain a retinal tissue marker, Chx10 (anti-ChxlO antibody, Exalpha Biologicals, sheep) and a photoreceptor precursor cell marker Crx (anti-Crx antibody, Takara Bio Inc., rabbit). The nuclei of the cells were stained with DAPI. These sections stained were observed by a fluorescence microscope (manufactured by KEYENCE CORPORATION.) to obtain immunostained images (Figure 5 and Figure 6). Further, whether a continuous epithelium structure of the neural retinal tissue was formed or not was checked in the same manner as in Example 1.

As a result, in cell aggregates preserved in preservation condition A (preservation condition in ordinary culture) and preserved in preservation conditions B, C and E and subjected to recovery culture, it was observed that the layer-like continuous epithelium structure of neural tissue is present in almost all of the cell aggregates. In preservation conditions D and F, the layer-like continuous epithelium structure of neural tissue was observed in some of the cell aggregates; however, a rosette structure was formed in a large number of cell aggregates (about 70%), that is, the form of the cell aggregate (three-dimensional retina) collapsed, as shown in Figure 5 and Figure 6. The cell aggregates on average showed the forms represented by D and I in Figure 5 and Figure 6. In contrast, in the cases of preservation conditions G and H using UW solution, it was found that the three-dimensional form of the retina tends to significantly collapse in almost all of the cell aggregates as shown in images indicated by G and H in Figure 5 and Figure 6.

From these results, it was found that a cell aggregate can be preserved for 48 hours while maintaining the continuous epithelium structure of neural retinal tissue in the condition of a preservation temperature of 17°C in various preservation solutions in preservation conditions B, C and E, compared to preservation condition in ordinary culture, i.e., preservation conditions A.

In contrast, in preservation conditions G and H in which UW solution was used as the preservation solution, it was found that the continuous epithelium structure of neural retinal tissue is rarely maintained.

### Example 3: Preservation of cell aggregate containing retinal tissue prepared from human iPS cells in Optisol as the preservation solution at 17°C

Human iPS cells (1231A3 strain, obtained from Kyoto University) were cultured in accordance with the method described in Example 1 in the feeder-free conditions to induce differentiation thereof and then subjected to suspension culture. Cell aggregates on Day 20 from initiation of the suspension culture were obtained.

The resultant cell aggregates on Day 20 from initiation of the suspension culture were cultured in serum mediums [1], [2] and [3] in accordance with the method of Example 2 up to Day 97 from initiation of the suspension culture. The resultant cell aggregates on Day 97 from initiation of the suspension culture were observed by an inverted microscope (EVOS, manufactured by Thermo Fisher Scientific K.K.). As a result, it was found that a continuous epithelium structure of neural tissue is contained.

Cell aggregates on Day 97 from initiation of the suspension culture were preserved in the following preservation conditions A to C.
- Preservation condition A: the preservation solution is Optisol, the preservation temperature is 17°C, the preservation container is a low adhesive plate (5% CO₂) and the preservation time is 48 hours
- Preservation condition B: the preservation solution is Optisol, the preservation temperature is 17°C, the preservation container is a low adhesive plate (5% CO₂) and the preservation time is 72 hours
- Preservation condition C: the preservation solution is Optisol, the preservation temperature is 17°C, the preservation container is a low adhesive plate (5% CO₂) and the preservation time is 120 hours

The cell aggregates preserved in preservation conditions A to C were subjected to recovery culture performed in medium B for 7 days in accordance with the method described in Example 2.

Each of the cell aggregates obtained after recovery culture was fixed with 4% paraformaldehyde, frozen and sectioned. The frozen sections obtained were subjected to immunostaining to stain a retinal tissue marker, Chx10 (anti-ChxlO antibody, Exalpha Biologicals, sheep) and a photoreceptor precursor cell marker Crx (anti-Crx antibody, Takara Bio Inc., rabbit), a photoreceptor marker Recoverin (anti-Recoverin antibody, Proteintech Group, rabbit), a retinal tissue marker Rx (anti-Rx antibody, Takara Bio Inc., guinea pig) and a proliferating cell marker Ki67 (anti-Ki67 antibody, BD, mouse). The nuclei of the cells were stained with DAPI. These sections stained were observed by a fluorescence microscope (manufactured by KEYENCE CORPORATION.) to obtain immunostained images (Figure 7 and Figure 8). Further, whether a continuous epithelium structure of the neural retinal tissue is formed or not was checked.

As a result, in cell aggregates preserved in any one of preservation conditions A to C, it was found that the continuous epithelium structure of neural tissue is highly frequently present. In the continuous epithelium structure of neural tissue in any one of preservation conditions A to C, it was found that Crx-positive photoreceptor precursor cells are present on the surface (apical surface side) and Chx10-positive neural retinal precursor cells are present inside them. From analysis of a series of sections, it was found that a Chx10-positive cell layer and a Ki67 positive cell layer are overlapped. It was further found that in the epithelium structure of neural tissue obtained in any one of preservation condition A to C, Recoverin-positive photoreceptor cells are present in the surface (apical surface side).

From these results, it was demonstrated that the continuous epithelium structure of neural tissue having a photoreceptor layer containing Crx-positive photoreceptor precursor cells together with Recoverin-positive photoreceptor cells on the surface (apical surface side) of a cell aggregate; a neural retinal precursor cell layer containing Chx10-positive neural retinal precursor cells together with Ki67-positive proliferating cells inside the photoreceptor layer; and a Crx-positive photoreceptor cells further inside the neural retinal precursor cell layer, i.e., a basal side, can be maintained, in conditions of a preservation temperature of 17°C and a preservation period of any one of 48 hours, 72 hours and 120 hours.

### Example 4: Preservation of cell aggregate containing retinal tissue prepared from human iPS cells in the presence or absence of 5% CO₂

Human iPS cells (1231A3 strain, obtained from Kyoto University) were cultured in accordance with the method described in Example 1 in the feeder-free conditions. As the feeder-free medium, StemFit medium (AK03N, manufactured by AJINOMOTO CO., INC.) was used. As the feeder-free scaffolding, Laminin 511-E8 (manufactured by Nippi. Inc.) was used.

Operation of differentiation induction was carried out as follows: Human iPS cells (1231A3 strain) were cultured in feeder-free StemFit medium up to the day before the cells reached sub-confluency (about 50% of the culture area is covered by cells). The human iPS cells the day before the cells reached sub-confluency were subjected to feeder-free culture for one day (preconditioning treatment) in the presence of SB431542 (5 µM) and SAG (300 nM)

The human iPS cells preconditioned were treated with TrypLE Select (manufactured by Life Technologies) and (further) separated into single cells by pipetting. Thereafter, the separated human iPS single cells were suspended in 100 µl of a serum-free medium such that cells per well of a non-cell adhesive 96-well culture plate (PrimeSurface, 96 V-bottom plate, manufactured by Sumitomo Bakelite Co., Ltd.) was 1.3 × 10⁴ cells and subjected to suspension culture in the conditions of 37°C and 5% CO₂. The serum-free medium (gfCDM + KSR) used herein was a serum-free medium prepared by adding 10% KSR and 450 µM 1-monothioglycerol and IX Chemically defined lipid concentrate to a mixture of culture fluids containing F-12 medium and IMDM medium in a ratio of 1:1. At the initiation time of the suspension culture (Day 0 from initiation of the suspension culture), IWR-1e (final concentration 3 µM), Y27632 (final concentration 20 µM) and SAG (final concentration 30 nM) were added to the serum-free medium. Day 3 from initiation of the suspension culture, 50 µl of a fresh serum-free medium (the same one as mentioned above) was added such that the final concentration of exogenous human recombinant BMP4 was 1.5 nM (55 ng/ml), and the final concentration of IWR-1e was 3 µM in a medium not containing Y27632 or SAG and containing IWR-1e and human recombinant BMP4 (manufactured by R&D).

Three days later (Day 6 from initiation of the suspension culture), 60 µl of the medium was discarded and 90 µl of the serum free medium not containing Y27632 or human recombinant BMP4 and containing IWR-1e (final concentration 3 µM) was added. The total volume of the medium was 180 µl.

Four days later (Day 10 from initiation of the suspension culture), 67% of the medium was exchanged with the serum-free medium, which did not contain IWR-1e, Y27632 or human recombinant BMP4. This medium exchange operation was repeated twice such that the concentration of exogenous IWR-1e becomes 10% or less compared to medium exchange.. Thereafter, a half of the medium was exchanged with the serum-free medium, which did not contain IWR-1e, Y27632 or human recombinant BMP4 once every 2 to 4 days. Operation for exchanging a half volume of the medium was carried out as follows. A half volume, i.e., 90 µl of the medium in the incubator was discarded and 90 µl of a fresh serum-free medium (the same one as mentioned above) was added. The total volume of the medium was 180 µl.

The cell aggregate obtained on Day 22 from initiation of the suspension culture was cultured in a serum-free medium (DMEM/F12 medium containing 1% N2 supplement) containing CHIR99021 (3 µM) and SU5402 (5 µM) for 3 days, i.e., up to Day 25 from initiation of the suspension culture.

The cell aggregate obtained on Day 25 from initiation of the suspension culture was cultured in serum mediums [1], [2] and [3] in accordance with the method described in Example 2 up to Day 66 from initiation of the suspension culture. The cell aggregates on Day 66 from initiation of the suspension culture were observed by an inverted microscope (EVOS, manufactured by Thermo Fisher Scientific K.K.). As a result, it was found that the continuous epithelium structure of neural tissue is contained

Using the cell aggregates obtained on Day 66 from initiation of the suspension culture, preservation conditions were studied.

The preservation solutions studied herein were all Optisol (described in Example 1).

The following two conditions including preservation temperature and surrounding conditions were studied.
- 17°C: incubator (5% CO₂ condition, manufactured by ASTEC Co., Ltd.)
- 17°C: centrifuge (atmospheric pressure, sealed condition, manufactured by Hitachi, Ltd.)

The following two preservation containers were studied.
- 60-mm low adhesive plate (low adhesive plate, manufactured by Sumitomo Bakelite Co., Ltd.)
- 2.0 mL CryoVial (manufactured by Thermo Fisher Scientific K.K.)

The following preservation conditions A and B were specifically studied.
- Preservation condition A: the preservation solution is Optisol, the preservation temperature is 17°C (incubator), the preservation container is a low adhesive plate (5% CO₂)
- Preservation condition B: the preservation solution is Optisol, the preservation temperature is 17°C (centrifuge, manufactured by Hitachi, Ltd.), the preservation container is a 2.0 mL-CryoVial (sealed in atmospheric pressure). In the conditions, in order to control temperature, the centrifuge was used without spinning. It was confirmed that the temperature within the centrifuge in the preservation period was maintained at 16.2 to 17.8°C, based on measurement by a temperature logger.

The cell aggregates on Day 66 from initiation of the suspension culture were preserved in the above preservation conditions A and B for 72 hours. The cell aggregates were observed immediately after completion of preservation by an inverted microscope (ECLIPSE Ti, manufactured by Nikon Corporation) as a bright field image (Figure 9, upper stage).

As a result, it was found that in cell aggregates observed immediately after completion of preservation in preservation condition A and preservation condition B, three-dimensional tissue form tends to be maintained at the same frequency.

Next, cell aggregates obtained after preservation in preservation conditions A and B were subjected to suspension culture performed in the medium B described in Example 2 by use of non-cell adhesive 96-well culture plate (PrimeSurface 96 U-bottom plate, manufactured by Sumitomo Bakelite Co.,) in an incubator at conditions of 37°C and 5% CO₂ (recovery culture).

Cell aggregates on Day 7 after initiation of recovery culture were observed by an inverted microscope (ECLIPSE Ti, manufactured by Nikon Corporation) as a bright field image (phase contrast image) (Figure 9, lower stage).

As a result, a layer-like continuous epithelium structure was highly frequently observed in cell aggregates preserved in preservation conditions A and B and subjected to recovery culture. The cell aggregates were observed by an inverted microscope (ECLIPSE Ti, manufactured by Nikon Corporation) as a Hoffman interference image. As a result, it was found that neural retinal tissue having a layer-like continuous epithelium structure is present at a high frequency in either one of preservation conditions A and B.

From these results, it was found that in the preservation condition B, where the tissue was preserved in a sealed condition in the air, three-dimensional neural tissue can be preserved for 72 hours while maintaining a continuous epithelium structure at a level that is not substantially different from the preservation condition A of 5% CO₂.

### Example 5: Measurement of survival rate of cells after cell aggregate containing retinal tissue prepared from human iPS cells was preserved in Optisol as the preservation solution at 17°C

Human iPS cells (1231A3 strain, obtained from Kyoto University) were cultured in accordance with the method described in Example 1 in the feeder-free conditions, and then induced differentiation thereof followed by suspension culture. A cell aggregate on Day 20 from initiation of the suspension culture was obtained.

The resultant cell aggregate on Day 20 from initiation of the suspension culture was cultured in serum mediums [1], [2] and [3] in accordance with the method described in Example 2 up to Day 91 from initiation of the suspension culture. Thereafter, the cell aggregate was prepared in the following condition A (preservation was not carried out) and condition B (preservation was carried out).
- Condition A (preservation was not carried out): Two cell aggregates on Day 91 from initiation of the suspension culture were cultured in medium B as the medium in the conditions of 37°C and 5% CO₂ for 72 hours.
- Condition B (preservation was carried out): Two cell aggregates on Day 91 from initiation of the suspension culture were preserved in Optisol as a preservation solution in an incubator (manufactured by WAKENBTECH CO., LTD.) as a preservation apparatus at 17°C in atmospheric pressure (sealed) for 72 hours.

The cell aggregates thus conditioned in condition A (preservation was not carried out) and condition B (preservation was carried out) each were dissociated by use of a neuronal cell dissociation solution (papain, manufactured by FUJIFILM Wako Pure Chemical Corporation) to prepare cell suspensions. The cell suspensions were subjected to measurement by NucleoCounter (manufactured by ChemoMetec A/S) to obtain survival rates of cells (Figure 10).

As a result, the survival rates of cells of two cell aggregates in condition A (preservation was not carried out) were 99.8% and 99.1%, respectively. In contrast, the survival rates of cells of two cell aggregates in condition B (preservation was carried out) were 99.3% and 98.2%, respectively. The results mean that the cells of cell aggregates even preserved exhibit an extremely high survival rate of 98% or more. From this, it was found that the survival rate of cells does not substantially differ between cell aggregates preserved and not-preserved.

From these results, it was demonstrated that there is no substantial difference in survival rate of cells between neural tissue preserved and neural tissue not preserved.

### Example 6: Study (1) Preservation temperature of cell aggregate containing retinal tissue prepared from human iPS cells

Human iPS cells (1231A3 strain, obtained from Kyoto University) were cultured in accordance with the method described in Example 4 in the feeder-free conditions (preconditioned), and then induced differentiation thereof. To the differentiated cells, CHIR99021 and SU5402 were applied. A "cell aggregate on Day 25 from initiation of the suspension culture" was obtained.

The cell aggregate obtained on Day 25 from initiation of the suspension culture was cultured in serum mediums [1], [2] and [3] described in Example 2 in 5% CO₂ condition up to Day 73 from initiation of the suspension culture. Thereafter, the cell aggregates on Day 73 from initiation of the suspension culture were observed by an inverted microscope (EVOS, manufactured by Thermo Fisher Scientific K.K.). As a result, it was found that the continuous epithelium structure of neural tissue is contained.

Using the cell aggregates on Day 73 from initiation of the suspension culture, preservation conditions were studied.

The preservation solutions studied herein were all Optisol (described in Example 1).

The following 5 preservation temperatures were studied.
- 4°C: refrigerator (sealed, manufactured by Panasonic Corporation)
- 8°C: centrifuge (sealed, manufactured by Hitachi, Ltd.)
- 17°C: centrifuge (sealed, manufactured by Hitachi, Ltd.)
- 30°C: thermostatic chamber (sealed, manufactured by AS ONE Corporation)
- 37°C: incubator (sealed or 5% CO₂, manufactured by Panasonic Corporation)
- The following two preservation containers were studied.
- 60-mm low adhesive plate (low adhesive plate, manufactured by Sumitomo Bakelite Co., Ltd.)
- 2.0 mL CryoVial (manufactured by Thermo Fisher Scientific K.K.)

The following preservation conditions A to F were specifically studied.
- Preservation condition A (preservation condition in ordinary culture):
   the preservation solution is B medium, the preservation temperature is 37°C, the preservation container is a low adhesive plate (5% CO₂)
- Preservation condition B: the preservation solution is Optisol, the preservation temperature is 4°C, the preservation container is 2.0 mL CryoVial (sealed)
- Preservation condition C: the preservation solution is Optisol, the preservation temperature is 8°C, the preservation container is 2.0 mL CryoVial (sealed)
- Preservation condition D: the preservation solution is Optisol, the preservation temperature is 17°C, the preservation container is 2.0 mL CryoVial (sealed)
- Preservation condition E: the preservation solution is Optisol, the preservation temperature is 30°C, the preservation container is 2.0 mL CryoVial (sealed)
- Preservation condition F: the preservation solution is Optisol, the preservation temperature is 37°C, the preservation container is 2.0 mL CryoVial (sealed)

Cell aggregates on Day 73 from initiation of the suspension culture were preserved in the preservation conditions A to F for 72 hours. The cell aggregates were observed immediately after completion of preservation by an inverted microscope (ECLIPSE Ti, manufactured by Nikon Corporation) as a bright field image (phase contrast image) (Figure 11, upper stage).

As a result, in almost all of the cell aggregates immediately after completion of preservation in preservation condition A (preservation condition in ordinary culture) and preservation condition D, it was found that three-dimensional tissue form was maintained. In preservation conditions B, C and E, some of cell aggregates maintained three-dimensional tissue form; however the form collapsed in a large number of cell aggregates. In contrast, in preservation condition F, three-dimensional tissue form significantly collapsed in almost all of the cell aggregates.

As recovery culture treatment, cell aggregates preserved in preservation conditions A to F were subjected to suspension culture performed in medium B described in Example 2 using a non-cell adhesive 96-well culture plate (PrimeSurface 96 U-bottom plate, manufactured by Sumitomo Bakelite Co., Ltd.) in an incubator in the conditions of 37°C and 5% CO₂ (recovery culture).

The cell aggregates obtained after the recovery culture were observed by an inverted microscope (ECLIPSE Ti, manufactured by Nikon Corporation) as a bright field image (phase contrast image) (Figure 11, middle stage).

As a result, in almost all of the cell aggregates preserved in preservation conditions A (preservation condition in ordinary culture) and D and subjected to recovery culture, neural tissue having a layer-like continuous epithelium structure was observed. In cell aggregates preserved in preservation conditions B, C and E and subjected to recovery culture, neural tissue having a layer-like continuous epithelium structure was observed some of cell aggregates; however, the form of cell aggregates (three-dimensional retina) collapsed in about 50% of the cell aggregates, as shown in the figure. The cell aggregates on average, showed forms indicated by B, C and E as shown in Figure 11. In contrast, in the case of preservation condition F, as shown in Figure 11, it was found that three-dimensional tissue form tends to significantly collapse in almost all of the cell aggregates.

Each of the cell aggregates obtained after recovery culture was fixed with 4% paraformaldehyde, frozen and sectioned. The frozen sections obtained were subjected to immunostaining to stain a photoreceptor precursor cell marker Crx (anti-Crx antibody, Takara Bio Inc., rabbit). These sections stained were observed by a fluorescence microscope (manufactured by KEYENCE CORPORATION.) to obtain immunostained images (Figure 11, lower stage). Then, whether Crx-positive photoreceptor precursor cells are present like a layer on the surface layer and the continuous epithelium structure of neural retinal tissue is present were checked in accordance with the method described in Example 1.

As a result, in cell aggregates obtained in preservation condition A (preservation condition in ordinary culture) and preservation condition D and subjected to recovery culture, it was found that the continuous epithelium structure of neural retinal tissue is present in almost all of the cell aggregates. In contrast, in preservation conditions B, C and E, the continuous epithelium structure of neural retinal tissue was observed in some of the cell aggregates; however, the three-dimensional form of the retina collapsed in about 50% of the cell aggregates, as shown in the figure. In preservation condition F, Crx-positive photoreceptor precursor cells are not present on the surface layer, no continuous epithelium structure was present, and rosette was formed in almost all of the cell aggregates.

Further, the number of Crx-positive cells in each of the cases of preservation conditions A to E was counted. In determining the number of the Crx-positive cells, an image having a size of 400×400 pixels was cut out from the image having Crx-positive cells per cell aggregate and magnified 20X by an inverted microscope (BZ-X710, manufactured by KEYENCE CORPORATION.) and photographed. The numbers of the positive cells in the whole image were compared (Figure 12). In the case of preservation condition F, a large number of Crx-positive photoreceptor precursor cells were observed; however, these cells were not present on the surface layer, a continuous epithelium structure was not observed and the form of cell aggregates significantly collapsed. Because of this, the number of the positive cells was not checked. As a result, it was found that the number of Crx-positive photoreceptor precursor cells in the cell aggregate preserved in preservation condition D is larger than that in the case of preservation condition A. In contrast, the numbers of Crx-positive photoreceptor precursor cells in the cell aggregates preserved in preservation conditions B, C and E were lower than that in the case of preservation condition A.

From these results, it was found that three-dimensional tissue form is maintained in almost all of the cell aggregates preserved in preservation condition D, more specifically, preserved at a preservation temperature of 17°C. In contrast, in preservation conditions B, C and E, more specifically, in the cell aggregates preserved at preservation temperatures of 4, 8 and 30°C, respectively, it was found that a three-dimensional tissue form was maintained in some of the cell aggregates; however, a large number of cell aggregates collapsed in form. It was also found that in the case of preservation condition F, more specifically, in the cell aggregates preserved at a preservation temperature of 37°C, three-dimensional tissue form significantly collapsed in almost all of the cell aggregates.

### Example 7: Study (2) Preservation temperature of cell aggregate containing retinal tissue prepared from human iPS cells

Human iPS cells (1231A3 strain, obtained from Kyoto University) were cultured in accordance with the method described in Example 4 in the feeder-free conditions and preconditioned to induce differentiation thereof. To the differentiated cells, CHIR99021 and SU5402 were applied to obtain "cell aggregate on Day 25 from initiation of the suspension culture".

The cell aggregate on Day 25 from initiation of the suspension culture was cultured in serum mediums [1], [2] and [3] described in Example 2 in 5% CO₂ condition up to Day 80 from initiation of the suspension culture. Thereafter, the cell aggregates on Day 80 from initiation of the suspension culture were observed by an inverted microscope (EVOS, manufactured by Thermo Fisher Scientific K.K.). As a result, it was found that the continuous epithelium structure of neural tissue is contained.

Using the cell aggregate on Day 80 from initiation of the suspension culture, preservation conditions were studied.

The preservation solutions studied herein were all Optisol (described in Example 1).

The following 5 preservation temperatures were studied.
- 4°C: refrigerator (sealed, manufactured by Panasonic Corporation)
- 12°C: centrifuge (sealed, manufactured by Hitachi, Ltd.)
- 17°C: centrifuge (sealed, manufactured by Hitachi, Ltd.)
- 22°C: centrifuge (sealed, manufactured by Hitachi, Ltd.)
- 37°C: incubator (sealed or 5% CO₂, manufactured by Panasonic Corporation)
- The following two preservation containers were studied.
- 60-mm low adhesive plate (low adhesive plate, manufactured by Sumitomo Bakelite Co., Ltd.)
- 2.0 mL CryoVial (manufactured by Thermo Fisher Scientific K.K.)

The following preservation conditions A to F were specifically studied.
- Preservation condition A (preservation condition in ordinary culture): the preservation solution is B medium, the preservation temperature is 37°C, the preservation container is a low adhesive plate (5% CO₂)
- Preservation condition B: the preservation solution is Optisol, the preservation temperature is 4°C, the preservation container is 2.0 mL CryoVial (sealed)
- Preservation condition C: the preservation solution is Optisol, the preservation temperature is 12°C, the preservation container is 2.0 mL CryoVial (sealed)
- Preservation condition D: the preservation solution is Optisol, the preservation temperature is 17°C, the preservation container is 2.0 mL CryoVial (sealed)
- Preservation condition E: the preservation solution is Optisol, the preservation temperature is 22°C, the preservation container is 2.0 mL CryoVial (sealed)
- Preservation condition F: the preservation solution is Optisol, the preservation temperature is 37°C, the preservation container is 2.0 mL CryoVial (sealed)

Cell aggregates on Day 80 from initiation of the suspension culture were preserved in the preservation conditions A to F for 72 hours. The cell aggregates were observed immediately after completion of preservation by an inverted microscope (ECLIPSE Ti, manufactured by Nikon Corporation) as a bright field image (phase contrast image) (Figure 13, upper stage).

As a result, it was found that three-dimensional tissue form is maintained in almost all of the cell aggregates immediately after completion of preservation in preservation condition A (preservation condition in ordinary culture) and preservation conditions C, D and E. In preservation condition B, some of cell aggregates maintained three-dimensional tissue form; however a large number of cell aggregates collapsed in form. In contrast, in preservation condition F, three-dimensional tissue form significantly collapsed in almost all of the cell aggregates.

Next, cell aggregates preserved in preservation conditions A to F were subjected to suspension culture performed in medium B described in Example 2 using a non-cell adhesive 96-well culture plate (PrimeSurface 96 U-bottom plate, manufactured by Sumitomo Bakelite Co., Ltd.) in an incubator in the conditions of 37°C and 5% CO₂ (recovery culture).

The cell aggregates obtained after the recovery culture were observed by an inverted microscope (ECLIPSE Ti, manufactured by Nikon Corporation) as a bright field image (phase contrast image) (Figure 13, middle stage).

As a result, neural tissue having a layer-like continuous epithelium structure was observed in almost all of the cell aggregates preserved in preservation condition A (preservation condition in ordinary culture) and preservation conditions C, D and E and subjected to recovery culture. In the case of preservation condition B, neural tissue having a layer-like continuous epithelium structure was observed is some of the cell aggregates; however, the form of cell aggregate (three-dimensional retina) collapsed in about 50% of the cell aggregates, as shown in Figure 13. In contrast, in the case of preservation condition F, as shown in Figure 13, F, it was found that form of three-dimensional tissue tends to significantly collapse in almost all of the cell aggregates.

Each of the cell aggregates obtained after recovery culture was fixed with 4% paraformaldehyde, frozen and sectioned. The frozen sections obtained were subjected to immunostaining to stain a photoreceptor precursor cell marker Crx (anti-Crx antibody, Takara Bio Inc., rabbit). These sections stained were observed by a fluorescence microscope (manufactured by KEYENCE CORPORATION.) to obtain immunostained images (Figure 13, lower stage). Then, whether Crx-positive photoreceptor precursor cells are present like a layer on the surface layer and whether the continuous epithelium structure of neural retinal tissue is present were checked in accordance with the method described in Example 1.

As a result, it was found that the continuous epithelium structure of neural retinal tissue is present in almost all of the cell aggregates preserved in preservation condition A (preservation condition in ordinary culture) and preservation conditions C, D and E, and subjected to recovery culture (Figure 13, lower stage). In contrast, in preservation condition B, the continuous epithelium structure of neural retinal tissue was observed in some of the cell aggregates; however, the three-dimensional form of the retina collapsed in about 50% of the cell aggregates, as shown in the figure. In preservation condition F, Crx-positive photoreceptor precursor cells are not present on the surface layer and rosette was formed in almost all of the cell aggregates.

Further, the number of Crx-positive photoreceptor precursor cells in each of preservation conditions A to E was counted. In determining the number of the Crx-positive cells, an image having a size of 400×400 pixels was cut out from the image having Crx-positive cells per cell aggregate and magnified 20X by an inverted microscope (BZ-X710, manufactured by KEYENCE CORPORATION.) and photographed. The numbers of the positive cells in the whole image were compared (Figure 14). In the case of preservation condition F, a large number of Crx-positive photoreceptor precursor cells were observed; however, these cells were not present on the surface layer, and the form of cell aggregates significantly collapsed. Because of this, the number of the positive cells was not checked. As a result, it was found that the numbers of Crx-positive photoreceptor precursor cells in cell aggregates preserved in preservation conditions D and E are larger than that of preservation condition A. In contrast, the number of Crx-positive photoreceptor precursor cells in the cell aggregate preserved in preservation condition C was almost the same as that of the cell aggregate preserved in preservation condition A. The number of Crx-positive photoreceptor precursor cells in the cell aggregate preserved in preservation condition B was lower than that in the case of preservation condition A.

From these results, layer-like three-dimensional tissue form was observed in almost all of the cell aggregates preserved in preservation conditions D and E, more specifically, preserved at a preservation temperature of 17°C and 22°C, respectively. In the cell aggregate preserved in preservation condition C, more specifically, preserved at a preservation temperature of 12°C, a continuous epithelium structure was observed at the same frequency as in the cases of preservation temperature of 17°C and 22°C; however, the number of photoreceptor precursor cells was low compared to the cases of 17 and 22°C. In the cell aggregate preserved in preservation condition B, more specifically, preserved at a preservation temperature of 4°C, the frequency of observing a continuous epithelium structure was low. In the case of preservation condition F, more specifically, at a preservation temperature of 37°C, it was found that not only continuous epithelium structure but also a cell aggregate itself tend to collapse. In short, it was found that 17 to 22°C are most preferable as the preservation temperature for neural tissue, and a preservation temperature of 12°C is also preferable.

### Example 8: Study of potassium ion concentration using a cell aggregate containing retinal tissue prepared from human iPS cells

The UW solution mentioned above is a preservation solution actually used in clinical sites for organ transplantation of endoderm tissue such as pancreatic islets and used as a standard in the transplantation industry. In contrast, it was suggested from the results of Example 2 that UW solution is unsuitable for preserving neural tissue. It is known that UW solution has a potassium chloride (KCl) concentration of 120 mM, which is higher than that of other preservation solutions (Table 6). Then, in order to find preferable conditions as the preservation solution, the effect of potassium chloride on preservation was checked.

Human iPS cells (1231A3 strain, obtained from Kyoto University) were cultured in accordance with the method described in Example 4 in the feeder-free conditions and preconditioned to induce differentiation thereof. To the differentiated cells, CHIR99021 and SU5402 were applied to obtain "cell aggregate on Day 25 from initiation of the suspension culture".

The cell aggregate on Day 25 from initiation of the suspension culture was cultured in serum mediums [1], [2] and [3] described in Example 2 in 5% CO₂ condition up to Day 99 from initiation of the suspension culture. Thereafter, the cell aggregate on Day 99 from initiation of the suspension culture was observed by an inverted microscope (EVOS, manufactured by Thermo Fisher Scientific K.K.). As a result, it was found that the continuous epithelium structure of neural tissue is contained.

Using the cell aggregate on Day 99 from initiation of the suspension culture, preservation conditions were studied.

The following 2 preservation solutions were studied.
- Optisol (described in Example 1)
- Optisol containing a high-concentration potassium ion (potassium chloride was added to Optisol so as to obtain a final potassium ion concentration of 120 mM)

The preservation temperatures studied were all 17°C (incubator, 5% CO₂, manufactured by ASTEC Co., Ltd.).

The preservation containers studied were all 60-mm low adhesive plate (low adhesive plate, manufactured by Sumitomo Bakelite Co., Ltd.).

The following preservation conditions A and B were specifically studied.
- Preservation condition A: the preservation solution is Optisol, the preservation temperature is 17°C, the preservation container is a low adhesive plate (5% CO₂)
- Preservation condition B: the preservation solution is Optisol containing high-concentration potassium ion, the preservation temperature is 17°C, the preservation container is a low adhesive plate (5% CO₂)

Cell aggregates on Day 99 from initiation of the suspension culture were preserved in the preservation conditions A and B for 120 hours. The cell aggregates were observed immediately after completion of preservation by an inverted microscope (ECLIPSE Ti, manufactured by Nikon Corporation) as a bright field image (phase contrast image) (Figure 15, upper stage).

As a result, it was found that three-dimensional tissue form is maintained in almost all of the cell aggregates immediately after completion of preservation in preservation condition A. In contrast, in preservation condition B, some of cell aggregates maintained three-dimensional tissue form; however a large number of cell aggregates collapsed in surface layer form.

Next, cell aggregates preserved in preservation conditions A and B were subjected to suspension culture performed in medium B described in Example 2 using a non-cell adhesive 96-well culture plate (PrimeSurface 96 U-bottom plate, manufactured by Sumitomo Bakelite Co., Ltd.) in an incubator in the conditions of 37°C and 5% CO₂ (recovery culture).

The cell aggregates obtained after the recovery culture were observed by an inverted microscope (ECLIPSE Ti, manufactured by Nikon Corporation) as a bright field image (phase contrast image) (Figure 15, middle stage).

As a result, in almost all of the cell aggregates preserved in preservation condition A and subjected to recovery culture, neural tissue having a layer-like continuous epithelium structure was highly frequently observed. In the case of preservation condition B, a three-dimensional structure of the retina tended to collapse in some of the cell aggregates.

Each of the cell aggregates obtained after recovery culture was fixed with 4% paraformaldehyde, frozen and sectioned. The frozen sections obtained were subjected to immunostaining to stain a photoreceptor precursor cell marker Crx (anti-Crx antibody, Takara Bio Inc., rabbit). These sections stained were observed by a fluorescence microscope (manufactured by KEYENCE CORPORATION.) to obtain immunostained images (Figure 15, lower stage). Then, whether the continuous epithelium structure of neural retinal tissue is present were checked in accordance with the method described in Example 6.

As a result, the continuous epithelium structure of neural retinal tissue was observed in almost all of the cell aggregates preserved in preservation condition A and subjected to recovery culture. In contrast, in the cell aggregates of preservation condition B, Crx-positive photoreceptor precursor cells were rarely observed. In addition, it failed to observe that these cells are present like a layer.

From these results, it was found that the three-dimensional form of the retina collapses and photoreceptor precursor cells die in a preservation solution having the same potassium ion concentration of 120 mM as in UW solution. More specifically, it can be demonstrated that a potassium ion concentration of a preservation solution for neural tissue of lower than 120 mM is important.

### Example 9: Preservation of cell aggregate containing retinal tissue prepared from human iPS cells

Human iPS cells (1231A3 strain, obtained from Kyoto University) were cultured in accordance with the method described in Example 4 in the feeder-free conditions and preconditioned to induce differentiation thereof. To the differentiated cells, CHIR99021 and SU5402 were applied to obtain "cell aggregate on Day 25 from initiation of the suspension culture".

The cell aggregate on Day 25 from initiation of the suspension culture was cultured in serum mediums [1], [2] and [3] described in Example 2 in 5% CO₂ condition up to Day 99 from initiation of the suspension culture. Thereafter, the cell aggregate on Day 99 from initiation of the suspension culture was observed by an inverted microscope (EVOS, manufactured by Thermo Fisher Scientific K.K.). As a result, it was found that the continuous epithelium structure of neural tissue is contained.

Using the cell aggregate on Day 99 from initiation of the suspension culture, preservation conditions were studied.

The following two preservation solution were studied (the composition of the individual preservation solutions are described in Table 7).
- DMEM, no glucose (manufactured by Thermo Fisher Scientific K.K.)
- DMEM, no glucose (manufactured by Thermo Fisher Scientific K.K. Scientific), to which a sodium pyruvate solution (manufactured by Thermo Fisher Scientific K.K.) was added so as to obtain a final concentration of 1 mM.

### [Table 7]

**Table 7 Components of DMEM, no glucose**

| Inorganic salts (concentration) | (mg/L) | (mM) |
|---|---|---|
| Calcium chloride (CaCl₂) (non-hydrate) | 200 | 1.80 |
| Iron nitrate Fe (NO₃)₃ 9H₂O | 0.100 | 0.000248 |
| Magnesium sulfate (MgSO₄) (non-hydrate) | 97.7 | 0.814 |
| Potassium chloride (KCl) | 400 | 5.33 |
| Sodium hydrogen carbonate (NaHCO₃) | 3700 | 44.0 |
| Sodium chloride (NaCl) | 6400 | 110 |
| Sodium dihydrogen phosphate (NaH₂PO₄ H₂O) | 125 | 0.906 |
| The other components contained | | |
| Phenol red, amino acid, vitamins | | |

(Excerpt from Thermo Fisher Scientific K.K. web page: https://www.thermofisher.com/jp/ja/home/technical-resources/media- fo rmulation.49.html)

The preservation temperatures studied were all 17°C (incubator, 5% CO₂, manufactured by ASTEC Co., Ltd.).

The preservation containers studied were all 60-mm low adhesive plate (low adhesive plate, manufactured by Sumitomo Bakelite Co., Ltd.).

More specifically, the following preservation conditions A and B were specifically studied.
- Preservation condition A: the preservation solution is DMEM, no glucose, the preservation temperature is 17°C, the preservation container is a low adhesive plate (5% CO₂)
- Preservation condition B: the preservation solution is DMEM, no glucose, to which sodium pyruvate solution was added so as to obtain a final concentration of 1 mM, the preservation temperature is 17°C, the preservation container is a low adhesive plate (5% CO₂).

Cell aggregate on Day 99 from initiation of the suspension culture were preserved in the preservation conditions A and B for 120 hours. The cell aggregates were observed immediately after completion of preservation by an inverted microscope (ECLIPSE Ti, manufactured by Nikon Corporation) as a bright field image (phase contrast image) (Figure 16, upper stage).

As a result, it was found that three-dimensional tissue form is maintained in almost all of the cell aggregates immediately after completion of preservation in both preservation conditions A and B.

Next, cell aggregates preserved in preservation conditions A and B were subjected to suspension culture performed in medium B described in Example 2 using a non-cell adhesive 96-well culture plate (PrimeSurface 96 U-bottom plate, manufactured by Sumitomo Bakelite Co., Ltd.) in an incubator in the conditions of 37°C and 5% CO₂ (recovery culture).

The cell aggregates obtained after the recovery culture were observed by an inverted microscope (ECLIPSE Ti, manufactured by Nikon Corporation) as a bright field image (phase contrast image) (Figure 16, middle stage).

As a result, layer-like continuous epithelium was highly frequently observed in almost all of the cell aggregates preserved in both preservation conditions A and B and subjected to recovery culture.

Each of the cell aggregates obtained after recovery culture was fixed with 4% paraformaldehyde, frozen and sectioned. The frozen sections obtained were subjected to immunostaining to stain a photoreceptor precursor cell marker Crx (anti-Crx antibody, Takara Bio Inc., rabbit). These sections stained were observed by a fluorescence microscope (manufactured by KEYENCE CORPORATION.) to obtain immunostained images (Figure 16, lower stage). Then, whether the continuous epithelium structure of neural retinal tissue is present was checked in accordance with the method described in Example 6.

As a result, the layer-like continuous epithelium structure of neural retinal tissue was observed in almost all of the cell aggregates preserved in both preservation conditions A and B and subjected to recovery culture.

From these results, it was found that if preservation is made at 17°C even in cell culture medium, DMEM, retinal tissue can be preserved without destroying its form and surface-layer continuous epithelium structure.

### Example 10: Preservation of cell aggregate containing fore/midbrain prepared from human iPS cells

Human iPS cells (1231A3 strain) were cultured in accordance with the method described in Example 1 in the feeder-free conditions in accordance with the method described in Scientific Reports, 4, 3594

(2014). As the feeder free medium, Stem Fit medium (AK03; manufactured by AJINOMOTO CO., INC.) was used. As a feeder-free scaffold, Laminin 511-E8 (manufactured by Nippi. Inc.) was used.

Operation of differentiation induction was carried out as follows: Human iPS cells (1231A3 strain) were cultured by using feeder-free StemFit medium until the day before the cells reached sub-confluency (about 50% of the culture area is covered by cells). The human iPS cells the day before the cells reached sub-confluency were subjected to feeder-free culture for one day (preconditioning treatment) in the presence of SB431542 (5 µM) and SAG (300 nM).

Preconditioned human iPS cells were treated with TrypLE Select (manufactured by Life Technologies) and (further) separated into single cells by pipetting. Thereafter, the separated human iPS single cells were suspended in 100 µl of a serum-free medium such that the density of cells per well of a non-cell adhesive 96-well culture plate (SUMILON spheroid, 96 V-bottom plate, manufactured by Sumitomo Bakelite Co., Ltd.) was 1.0×10⁴ cells and subjected to suspension culture in the conditions of 37°C and 5% CO₂. The serum-free medium (GMEM+KSR) used herein was a serum-free medium prepared by adding 20% KSR (manufactured by Life Technologies), 0.1 mM 2-mercaptoethanol, IX non-essential amino acids (manufactured by Life Technologies) and 1 mM pyruvic acid (manufactured by Life Technologies) to GMEM medium (manufactured by Life Technologies).

At the initiation of the suspension culture (Day 0 from initiation of the suspension culture), a Wnt signal transducing pathway inhibitor (IWR-1-endo, 3 µM), TGFβR inhibitor (SB431542, 5 µM) and Y27632 (20 µM) were added to the serum-free medium and the culture was initiated.

On Day 4 from initiation of the suspension culture, 80 µl of a serum-free medium not containing Y27632 and containing the Wnt signal transducing pathway inhibitor (IWR-1-endo, 3 µM) and TGFβR inhibitor (SB431542, 5 µM) were added. The total volume of the medium was controlled to be 180 µl. Thereafter, a half volume of the medium was exchanged with the serum-free medium not containing Y27632 and containing a Wnt signal transducing pathway inhibitor (IWR-1-endo, 3 µM) and TGFβR inhibitor (SB431542, 5 µM) once every 2 to 4 days from initiation of the suspension culture up to Day 17.

The cell aggregate obtained on Day 17 from initiation of the suspension culture was cultured in a serum-free medium (DMEM/F12 medium) containing 1% N2 supplement and 1% chemically defined lipid concentrate (manufactured by Thermo Fisher Scientific K.K.) in the condition of 5% CO₂ from initiation of the suspension culture up to Day 45 from initiation of the suspension culture. The cell aggregate on Day 45 was observed by an inverted microscope (EVOS, manufactured by Thermo Fisher Scientific K.K.). As a result, it was found that neuroepithelial tissue is contained.

Using the resultant cell aggregate on Day 45 from initiation of the suspension culture, preservation conditions were studied.

The following 4 preservation solutions were studied (compositions of individual preservation solutions are defined above).
- Serum-free medium (DMEM/F12 medium) containing 1% N2 supplement and 1% chemically defined lipid concentrate
- Optisol (described in Example 1)
- HBSS (described in Example 1)
- UW solution (described in Example 2)

The following two preservation temperatures were studied
- 37°C: incubator, 5% CO₂, manufactured by Panasonic Corporation
- 17°C: incubator, 5% CO₂, manufactured by ASTEC Co., Ltd.

The preservation containers used herein were all a 60-mm low adhesive plate (low adhesive plate, manufactured by Sumitomo Bakelite Co., Ltd.).

The following preservation conditions A to D were specifically studied:
- Preservation condition A (preservation condition in ordinary culture): the preservation solution is serum-free medium (DMEM/F12 medium) containing 1% N2 supplement and 1% chemically defined lipid concentrate, the preservation temperature is 37°C, the preservation container is a low adhesive plate (5% CO₂)
- Preservation condition B: the preservation solution is Optisol, the preservation temperature is 17°C, the preservation container is a low adhesive plate (5% CO₂)
- Preservation condition C: the preservation solution is HBSS, the preservation temperature is 17°C, the preservation container is a low adhesive plate (5% CO₂)
- Preservation condition D: the preservation solution is UW solution, the preservation temperature is 17°C, the preservation container is a low adhesive plate (5% CO₂)

The cell aggregates on Day 45 from initiation of the suspension culture were preserved in the above preservation conditions A to D for 72 hours. The cell aggregates were observed immediately after completion of preservation by an inverted microscope (ECLIPSE Ti, manufactured by Nikon Corporation) as a bright field image (phase contrast image), (Figure 17, upper stage). Neuroepithelial tissue was observed in cell aggregates immediately after completion of preservation in any one of the preservation conditions.

Next, cell aggregates preserved in preservation conditions A to D were subjected to suspension culture in serum-free medium (DMEM/F12 medium) containing 1% N2 supplement and 1% chemically defined lipid concentrate using a non-cell adhesive 96-well culture plate (PrimeSurface 96 U-bottom plate, manufactured by Sumitomo Bakelite Co., Ltd.) in an incubator in the conditions of 37°C and 5% CO₂ (recovery culture).

The cell aggregates obtained after the recovery culture were observed by an inverted microscope (ECLIPSE Ti, manufactured by Nikon Corporation) as a bright field image (phase contrast image) (Figure 17, middle stage).

As a result, neuroepithelial tissue was observed in some of cell aggregates preserved in preservation condition A (preservation condition in ordinary culture) and preservation conditions B and C and subjected to recovery culture. In contrast, in cell aggregates preserved in preservation condition D, a cell aggregate having neuroepithelial tissue was not observed at all.

Each of the cell aggregates obtained after recovery culture was fixed with 4% paraformaldehyde, frozen and sectioned. The frozen sections obtained were subjected to immunostaining to stain a photoreceptor precursor cell marker Rx (anti-Rx antibody, manufactured by Takara Bio Inc., guinea pig) and a forebrain and/or midbrain marker, Otx2 (anti-Otx2 antibody, manufactured by Abcam plc., rabbit). These sections stained were observed by a fluorescence microscope (manufactured by KEYENCE CORPORATION.) to obtain immunostained images (Figure 17, lower stage). The presence or absence of Rx-negative and Otx2-positive lumen structure (nerve rosette) formed of forebrain tissue and/or midbrain tissue, without containing retinal tissue was checked.

As a result, the lumen structure was observed in some of the cell aggregates preserved in preservation condition A (preservation condition in ordinary culture), and preservation conditions B and C and subjected to recovery culture. In contrast, the cell aggregate preserved in preservation condition D and subjected to recovery culture having a lumen structure was not observed at all.

The number of cell aggregates having a lumen structure formed of forebrain tissue and/or midbrain tissue without containing retinal tissue was checked (Figure 18).

As a result, the lumen structure was observed in 4 out of 6 cell aggregates preserved in preservation condition A (preservation condition in ordinary culture) and subjected to recovery culture. The lumen structure was observed in 2 out of 6 cell aggregates preserved in preservation condition B and subjected to recovery culture. The lumen structure was observed in 3 out of 6 cell aggregates preserved in preservation condition C and subjected to recovery culture. In contrast, a lumen structure was not observed in 6 cell aggregates preserved in preservation condition D and subjected to recovery culture.

From these results, it was found that a cell aggregate can be preserved in Optisol or HBSS as the preservation solution for 72 hours while maintaining cell aggregate form having a lumen structure constituted of forebrain and/or midbrain tissue in preservation conditions B and C where the preservation temperature is 17°C, unlike preservation condition A, which is the same preservation condition as the preservation condition in ordinary culture.

In contrast, in preservation condition D using UW solution, it was found that the lumen structure is rarely maintained.

The results were almost the same as the preservation results found in neural retinal tissue in Example 2.

From the results, it was found that, not only in the case of the neural tissue having a continuous epithelium structure as neuroepithelial structure but also in the case of fore/midbrain tissue having a lumen structure as a neuroepithelial structure, if the preservation temperature is about 17°C, the neural tissue can be preserved while maintaining the neuroepithelial structure. With respect to the composition of the preservation solution, the UW solution having a potassium ion concentration of 120 mM is not suitable even for preserving fore/midbrain tissue.

### Example 11: Transplantation of preserved cell aggregate containing retinal tissue prepared from human ES cells to retinal degenerative rat

Human ES cells (KhES1 strain, obtained from Kyoto University and used in RIKEN CENTER FOR DEVELOPMENTAL BIOLOGY) were cultured in accordance with the method described in Example 2 in the feeder-free conditions (preconditioned) to induce differentiation thereof. To the differentiated cells, CHIR99021 and SU5402 were applied. A "cell aggregate on Day 21 from initiation of the suspension culture" was obtained.

The cell aggregate on Day 21 from initiation of the suspension culture was cultured in serum mediums [1], [2] and [3] in accordance with the method described in Example 2 in the condition of 5% CO₂ up to Day 80 from initiation of the suspension culture.

The cell aggregate on Day 80 from initiation of the suspension culture was prepared in the following conditions A (preservation was not carried out) and B (preservation was carried out).
- Condition A (preservation was not carried out): cell aggregate on Day 80 from initiation of the suspension culture was cultured in medium B at 37°C for 48 hours in the condition of 5% CO₂.
- Condition B (preservation was carried out): cell aggregate on Day 80 from initiation of the suspension culture was preserved in Optisol used as a preservation solution in an incubator (5% CO₂ condition, manufactured by ASTEC Co., Ltd.) as a preservation apparatus at 17°C and cultured at 17°C for 48 hours in the condition of 5% CO₂.

The preservation containers used herein were all a 60-mm low adhesive plate (low adhesive plate, manufactured by Sumitomo Bakelite Co., Ltd.).

The cell aggregates obtained in condition A (preservation was not carried out) and condition B (preservation was carried out) were transplanted in the subretina of retinal degenerative nude rats (SD-Foxn1 Tg (S334ter)3LavRrrc nude rat), which is a photoreceptor degeneration model, by use of syringe in accordance with the method described in the literature (Shirai et al., PNAS 113, E81-E90).

The eye tissue obtained on Day 240 from initiation of the suspension culture was fixed with paraformaldehyde (PFA fixed) and subjected to sucrose replacement. The eye tissue fixed, frozen and sectioned by use of a cryostat. These frozen sections were subjected to immunostaining to stain a rod photoreceptor marker, Rhodopsin (anti-RetPl antibody, Sigma-Aldrich Co. LLC., mouse) and a cone photoreceptor precursor cell marker, S+M+L opsin (anti-S+M+ L opsin antibody, Merck Millipore, rabbit). Expression of Crx was observed in a Crx:: Venus fluorescent image. Fluorescent images of the frozen sections immunostained were observed by use of a confocal laser microscope (trade name: TCS SP8, manufactured by Leica Camera AG) (Figure 19).

First, in the rat, in the region where human cells derived from the transplanted cell aggregate are not present, photoreceptor cells were rarely observed. In the retina of the rat transplanted with the cell aggregate obtained in condition A (preservation was not carried out), it was observed that rod photoreceptor cells positive to Rhodopsin and cone photoreceptor cells positive to S+M+L opsin form a rosette structure, engrafted and matured. In the retina of the rat transplanted with the cell aggregate obtained in condition B (preservation was carried out), it was observed that rod photoreceptor cells positive to Rhodopsin and cone photoreceptor cells positive to S+M+L opsin form a rosette structure, engrafted and matured. According to microscopic observation of a region further wider than this, it was found that a photoreceptor rosette was engrafted at almost the same frequency and matured, not only in condition A (preservation was not carried out) but also in condition B (preservation was carried out).

From these results, it was found that even if preserved retinal tissue was transplanted, the tissue is engrafted and matured in substantially the same level as in the retinal tissue not preserved. In other words, it was successfully demonstrated that if the method according to the present invention is used, neural tissue suitable for transplantation can be preserved.

### Example 12: Concentration of chondroitin sulfate in cell mass containing retinal tissue prepared from human iPS cells

Human iPS cells (TFH-HA strain, established by Sumitomo Dainippon Pharma Co., Ltd.) are those established by using commercially available Sendai virus vector (4 factors, i.e., Oct3/4, Sox2, KLF4, c-Myc, site tune kit manufactured by ID Pharma Co., Ltd.) based on the method described in the protocol open to public by Thermo Fisher Scientific K.K. (iPS 2.0 Sendai Reprogramming Kit, Publication Number MAN0009378, Revision 1.0) and protocol open to public (establishment/maintenance culture of feeder-free human iPS cells, CiRA_Ff-iPSC_protocol_JP_v140310, http://www.cira.kyoto-u.ac.jp/j/research/protocol.html) by Kyoto University, and using StemFit medium (AK03; manufactured by AJINOMOTO CO., INC.) and Laminin 511-E8 (manufactured by Nippi. Inc.).

The human iPS cells (TFH-HA strain) were cultured in accordance with the method described in Example 1 in StemFit medium in feeder-free conditions until the day before the cells reached sub-confluency. The human iPS cells (TFH-HA strain) the day before the cells reached sub-confluent were cultured in the presence of SB431542 (5 µM) and SAG (300 nM) in feeder-free culture for one day (preconditioning treatment).

Preconditioned human iPS cells were treated with TrypLE Select (manufactured by Life Technologies) and (further) separated into single cells by pipetting. Thereafter, the separated human iPS single cells were suspended in 100 µl of a serum-free medium such that the density of cells per well of a non-cell adhesive 96-well culture plate (PrimeSurface, 96 V-bottom plate, manufactured by Sumitomo Bakelite Co., Ltd.) was 1.3×10⁴ cells and subjected to suspension culture in the conditions of 37°C and 5% CO₂. The serum-free medium (gfCDM + KSR) used herein was a mixture of culture fluids consisting of F-12 medium and IMDM medium in a ratio of 1:1 and containing 10% KSR and 450 µM 1-monothioglycerol and 1X Chemically defined lipid concentrate. At the initiation time of the suspension culture (Day 0 from initiation of the suspension culture), Y27632 (final concentration 20 µM) and SAG (final concentration 10 nM) were added to the serum-free medium. Day 3 from initiation of the suspension culture, 50 µl of a fresh serum-free medium (the same one as mentioned above) was added to a medium not containing Y27632 or SAG and containing human recombinant BMP4 (manufactured by R&D), such that the final concentration of exogenous human recombinant BMP4 was 1.5 nM (55 ng/ml).

Four days later (that is, Day 7 from initiation of the suspension culture), the medium was exchanged with the serum free medium not containing Y27632 or human recombinant BMP4. Operation for exchanging the medium was carried out as follows. First, 60 µl of the medium in the incubator was discarded, and then, 90 µl of a fresh serum-free medium (the same one as mentioned above) was added to control a total medium volume to be 180 µl. Thereafter, a half of the medium was exchanged with serum-free medium not containing Y27632 or human recombinant BMP4, once every 2 to 4 days. Operation for exchanging a half volume of the medium was carried out as follows. A half volume, i.e., 90 µl of the medium in the incubator was discarded, and then, 90 µl of a fresh serum-free medium (the same one as mentioned above) was added. The total volume of the medium was controlled to be 180 µl.

The cell mass obtained on Day 17 from initiation of the suspension culture was cultured in a serum-free medium (DMEM/F12 medium containing 1% N2 supplement) containing CHIR99021 (3 µM) and SU5402 (5 µM) for 3 days, i.e., up to Day 20 from initiation of the suspension culture.

The cell aggregate obtained on Day 20 from initiation of the suspension culture was cultured in serum mediums [1], [2] and [3] described below up to Day 84 from initiation of the suspension culture in the condition of 5% CO₂.
[1] Day 20 to Day 40 from initiation of the suspension culture: medium A.
[2] Day 40 to Day 60 from initiation of the suspension culture: mixture of culture fluids containing medium A and medium B in a ratio of 1:3.
[3] On and after Day 60 from initiation of the suspension culture: medium B.

The resultant cell aggregate on Day 84 from initiation of the suspension culture was observed by an inverted microscope (EVOS, manufactured by Thermo Fisher Scientific K.K.). As a result, it was found that a continuous epithelium structure of neural tissue is contained. Using the cell aggregate on Day 84 from initiation of the suspension culture, preservation conditions were studied.

The following 4 types of preservation solutions were studied (see Tables 4, 8 and 9).
- Medium B
- Optisol (containing 2.5% chondroitin sulfate)
- 1% chondroitin sulfate-containing medium (mixture of culture fluids containing DMEM, low glucose and DisCoVisc in a ratio of 3:1. DMEM, low glucose used herein was manufactured by Thermo Fisher Scientific K.K. (model number: 11885-084). DisCoVisc used herein was manufactured by Alcon Japan Ltd (sales name code: 1319818Q1025)
- 0.5% chondroitin sulfate-containing medium (mixture of culture fluids containing DMEM low glucose and DisCoVisc in a ratio of 7:1)

### [Table 8]

**Table 8 List of components of DMEM, low glucose**

| Inorganic salts (concentration) | (mg/L) | (mM) |
|---|---|---|
| Calcium chloride (CaCl₂) (non-hydrate) | 200 | 1.80 |
| Iron oxalate Fe(NO₃)₃9H₂O | 0.100 | 0.000248 |
| Magnesium sulfate (MgSO₄) (non-hydrate) | 97.7 | 0.814 |
| Potassium chloride (KCl) | 400 | 5.33 |
| Sodium hydrogen carbonate (NaHCO₃) | 3700 | 44.0 |
| Sodium chloride (NaCl) | 6400 | 110 |
| Sodium dihydrogen phosphate (NaH₂PO₄ H₂O) | 125 | 0.906 |
| The other components contained | | |
| Phenol red, D- glucose, sodium pyruvate | | |

### [Table 9]

**Table 9 Components of DisCoVisc**

| | (mg/mL) |
|---|---|
| Sodium hyaluronate | 16.5 |
| Sodium chondroitin sulfate | 40 |
| The other components contained | |
| Sodium dihydrogen phosphate hydrate (NaH₂PO₄ H₂O), anhydrous sodium monohydrogen phosphate (Na₂HPO₄), isotonic agent, two pH regulator components | |

The following two preservation temperatures were studied.
- 37°C (incubator, 5% CO₂ condition, manufactured by Panasonic Corporation)
- 17°C (thermostatic chamber, atmospheric pressure, sealed condition, manufactured by WAKENBTECH CO.,LTD.)

The following two preservation containers were studied.
60-mm low adhesive plate (low adhesive plate, manufactured by Sumitomo Bakelite Co., Ltd.)
2.0 mL CryoVial (manufactured by Thermo Fisher Scientific K.K.)

The following A to D preservation conditions were specifically studied.
- Preservation condition A: (preservation condition in ordinary culture): the preservation solution is B medium, the preservation temperature is 37°C (incubator), the preservation container is a 60-mm low adhesive plate (5% CO₂)
- Preservation condition B: the preservation solution is Optisol, the preservation temperature is 17°C (thermostatic chamber), the preservation container is 2.0 mL CryoVial (sealed at atmospheric pressure)
- Preservation condition C: the preservation container is 1% chondroitin sulfate-containing medium, the preservation temperature is 17°C (thermostatic chamber), the preservation container is a 2.0 ml CryoVial (sealed at atmospheric pressure)
- Preservation condition D: the preservation container is 0.5% chondroitin sulfate-containing medium, the preservation temperature is 17°C (thermostatic chamber), the preservation container is a 2.0 ml CryoVial (sealed at atmospheric pressure).

The cell aggregates on Day 84 from initiation of the suspension culture were preserved in the above preservation conditions A to D for 72 hours. The cell aggregates were observed immediately after completion of preservation by an inverted microscope (IX83, manufactured by Olympus Corporation) as a bright field image (phase contrast image), (Figure 20, upper stage). As a result, three-dimensional tissue form was maintained in almost all of the cell aggregates in any one of the preservation conditions.

Further, cell aggregates preserved in preservation conditions A to D were subjected to suspension culture performed in medium B using a non-cell adhesive 96-well culture plate (PrimeSurface 96 slit-well plate, manufactured by Sumitomo Bakelite Co., Ltd.) in an incubator in the conditions of 37°C and 5% CO₂ (recovery culture).

The cell aggregates obtained after the recovery culture were observed by an inverted microscope (IX83, manufactured by Olympus Corporation) as a bright field image (phase contrast image) (Figure 20, the second stage from the top). As a result, three-dimensional tissue form was maintained in almost all of the cell aggregates and neuroepithelial structure was observed in any one of the preservation conditions.

Each of the cell aggregates obtained after recovery culture was fixed with 4% paraformaldehyde, frozen and sectioned. The frozen sections obtained were subjected to immunostaining to stain a photoreceptor precursor cell marker Crx (anti-Crx antibody, Takara Bio Inc., rabbit) and Chx10 (anti-ChxlO antibody, Exalpha Biologicals, sheep). These sections stained were observed by a fluorescence microscope (manufactured by KEYENCE CORPORATION.) to obtain immunostained images (Figure 20, the second stage from the bottom and the bottom stage). Then, whether Crx-positive photoreceptor precursor cells are present like a layer on the surface layer and whether the continuous epithelium structure of neural retinal tissue is present, were checked in accordance with the method described in Example 1. As a result, it was found that the Crx-positive photoreceptor precursor cells are present on the surface layer like a layer (Figure 20, the second stage from the bottom). It was further found that the Crx10-positive photoreceptor precursor cells are present like a layer inside the Crx-positive cell layer on the surface layer (Figure 20, the bottom layer). From these results, it was found that the epithelium structure of neural retinal tissue is highly frequently present in any one of the preservation conditions.

From these results, it was found that three-dimensional tissue form is maintained in almost all of the cell aggregates in any one of the preservation conditions. More specifically, three-dimensional retina was successfully preserved in a solution having a chondroitin sulfate concentration of 0.5 to 2.5% at a low temperature.

Cell aggregates were successfully preserved by using HBSS in Example 1 and DMEM in Example 9 for 48 hours and 120 hours, respectively. Since these solutions do not contain chondroitin sulfate, it was demonstrated that the concentration of chondroitin sulfate maintaining the three-dimensional form of the retina is 0 to 2.5%.

### Example 13: Transplantation of preserved cell aggregate containing retinal tissue prepared from human iPS cells to retinal degenerative rat

Human iPS cells (DSP-SE strain, established by Sumitomo Dainippon Pharma Co., Ltd.) were those established by using Sendai virus vector (4 factors, i.e., Oct3/4, Sox2, KLF4, c-Myc, site tune kit manufactured by ID Pharma Co., Ltd.) based on the method described in the protocol open to public by Thermo Fisher Scientific K.K. (iPS 2.0 Sendai Reprogramming Kit, Publication Number MAN0009378, Revision 1.0) and protocol open to public (establishment/maintenance culture of feeder-free human iPS cells, CiRA_Ff-iPSC_protocol_JP_v140310, http://www.cira.kyoto-u.ac.jp/j/research/protocol.html) by Kyoto University, and using StemFit medium (AK03; manufactured by AJINOMOTO CO., INC.) and Laminin 511-E8 (manufactured by Nippi. Inc.).

The human iPS cells (DSP-SE strain) were cultured in accordance with the method described in Example 1 in StemFit medium in a feeder-free condition up to the day before the cells reached sub-confluency.

Operation of differentiation induction was carried out as follows: Human iPS cells (DSP-SE strain) on the day before the cells reached sub-confluency were cultured further for one day in a feeder-free condition in the presence of SB431542 (5 µM) and SAG (300 nM), (preconditioning treatment).

Preconditioned human iPS cells were treated with TrypLE Select (manufactured by Life Technologies) and (further) separated into single cells by pipetting. Thereafter, the separated human iPS single cells were suspended in 100 µl of a serum-free medium such that the density of cells per well of a non-cell adhesive 96-well culture plate (PrimeSurface, 96 V-bottom plate, manufactured by Sumitomo Bakelite Co., Ltd.) was 1.3×10⁴ cells and subjected to suspension culture in the conditions of 37°C and 5% CO₂. The serum-free medium (gfCDM+KSR) used herein was a serum-free medium prepared by adding 10% KSR, 450 µM 1-monothioglycerol, 1X Chemically defined lipid concentrate to a mixture of culture fluids of F-12 medium and IMDM medium in a ratio of 1:1. At the initiation time of the suspension culture (Day 0 from initiation of the suspension culture), IWR-1e (final concentration 3 µM), Y27632 (final concentration 20 µM) and SAG (final concentration10 nM) were added to the serum-free medium. Day 3 from initiation of the suspension culture, 50 µl of a fresh serum free medium (the same one as mentioned above) was added to a medium not containing Y27632 or SAG and containing human recombinant BMP4 (manufactured by R&D) and IWR-1e (final concentration 3 µM) such that the final concentration of exogenous human recombinant BMP4 became 1.5 nM (55 ng/ml).

Three days later (that is, Day 6 from initiation of the suspension culture), the medium was exchanged with the serum free medium not containing Y27632, SAG, human recombinant BMP4 or IWR-1e. Operation of exchanging medium was carried out as follows: First, 100 µl of the medium in the incubator was discarded such that the concentration of exogenous IWR-1e was 10% or less compared to the concentration before medium exchange. Then, 130 µl of a fresh serum-free medium (the same one as mentioned above) was added. Thereafter, 130 µl of the medium in the incubator was discarded, and 130 µl of a fresh serum-free medium (the same one as mentioned above) was added. The total volume of the medium was controlled to be 180 µl. Thereafter, a half of the medium was exchanged with the serum-free medium, which did not contain IWR-1e, Y27632, SAG or human recombinant BMP4, once every 2 to 4 days. Operation for exchanging a half volume of the medium was carried out as follows. A half volume, i.e., 90 µl, of the medium in the incubator was discarded, 90 µl of fresh serum-free medium (the same one as mentioned above) was added. The total volume of the medium was controlled to be 180 µl.

The cell mass obtained on Day 13 from initiation of the suspension culture was cultured in a serum-free medium (DMEM/F12 medium containing 1% N2 supplement) containing CHIR99021 (3 µM) and SU5402 (5 µM) for 3 days, i.e., up to Day 16 from initiation of the suspension culture.

The cell aggregate obtained on Day 16 from initiation of the suspension culture was cultured in serum mediums [1], [2] and [3] described below up to Day 82 from initiation of the suspension culture in the condition of 5% CO₂.
[1] Day 21 to Day 40 from initiation of the suspension culture: medium A.
[2] Day 40 to Day 60 from initiation of the suspension culture: mixed medium containing medium A and medium B in a ratio of 1:3.
[3] On and after Day 60 from initiation of the suspension culture: medium B.

The resultant cell aggregate on Day 82 from initiation of the suspension culture was preserved in Optisol as a preservation solution in a 17°C- thermostatic chamber (atmospheric pressure, sealed condition, manufactured by WAKENBTECH CO.,LTD.) used as a preservation apparatus at 17°C for 96 hours.

The preservation container studied herein was a 1.5 mL Eppendorf tube (Eppendorf AG).

The cell aggregates were transplanted in the subretina of retinal degenerative nude rats (SD-Foxn1 Tg (S334ter)3LavRrrc nude rat), which is a photoreceptor degeneration model, by use of syringe in accordance with the method described in the literature (Shirai et al., PNAS 113, E81-E90).

The eye tissue obtained on Day 294 from initiation of the suspension culture was fixed with paraformaldehyde (PFA fixed) and subjected to sucrose replacement. The fixed eye tissue was frozen and sectioned by use of a cryostat. These frozen sections were immunostained with a rod photoreceptor marker, Rhodopsin (anti-RetPl antibody, Sigma-Aldrich Co. LLC., mouse), a cone photoreceptor precursor cell marker, cone arrestin (anti-cone arrestin antibody, Novus Biologicals, goat), a photoreceptor outer segment marker, PRPH2 (anti-Peripherin-2 antibody, Proteintech Group, rabbit) and a human nucleus marker, human nuclei (anti-Ku80 antibody, R&D systems, Inc., goat). Fluorescent images of the frozen sections immunostained were observed by use of a confocal laser microscope (trade name: TCS SP8, manufactured by Leica Camera AG) (Figure 21, A to E).

First, in the region of a rat where human cells derived from the transplanted cell aggregate are not present, photoreceptor cells were rarely observed. In the retina of the rat transplanted with the cell aggregate preserved at 17°C for 96 hours, it was observed that rod photoreceptor cells positive to Rhodopsin forms a rosette structure and engrafted and matured. Further, Cone arrestin-positive cone photoreceptor cells and PRPH2-positive photoreceptor outer segment were detected. From this, it was found that the transplanted retinal tissue is engrafted and matured.

From these results, it was found that retinal tissue preserved for 96 hours at a low temperature and transplanted is engrafted and matured. More specifically, it was demonstrated that neural tissue suitable for transplantation can be preserved for 4 days if the method of the invention of the application is used.

### Industrial Applicability

According to the present invention, it is possible to preserve neural tissue excised out from a living body and prepared from pluripotent stem cells, for a period not more than two weeks without freezing. Owing to the method of the invention, treatment for a neurodegenerative disease by transplantation therapy can be more easily realized.

## Claims

1. A preservation method for neural tissue, comprising preserving neural tissue in a preservation solution having a potassium ion concentration of more than 0 mM and less than 115 mM at a preservation temperature of 8°C to 30°C.

2. The preservation method according to claim 1, wherein the preservation temperature is 12°C to 22°C.

3. The preservation method according to claim 1 or 2, wherein the neural tissue is derived from a human.

4. The preservation method according to any one of claims 1 to 3, wherein the neural tissue is a tissue derived from pluripotent stem cells.

5. The preservation method according to any one of claims 1 to 4, wherein the neural tissue is a tissue to be subjected to transplantation.

6. The preservation method according to any one of claims 1 to 5, wherein the neural tissue is a cell aggregate having a layer structure.

7. The preservation method according to claim 6, wherein the neural tissue is a cell aggregate having a neuroepithelial structure.

8. The preservation method according to claim 7, wherein the neuroepithelial structure is a continuous epithelium structure present on a surface of the cell aggregate and/or a rosette structure present on a surface of or inside the cell aggregate.

9. The preservation method according to any one of claims 1 to 8, wherein the neural tissue includes one or more neural tissues selected from the group consisting of forebrain tissue, midbrain tissue and retinal tissue.

10. The preservation method according to any one of claims 1 to 9, for preservation for a period of 14 days or less.

11. The preservation method according to any one of claims 1 to 9, for preservation for a period of 5 days or less.

12. The preservation method according to any one of claims 1 to 11, wherein the preservation solution is an aqueous solution containing a buffering agent and having a buffering action to control pH within the range of 6.0 to 8.6.

13. The preservation method according to any one of claims 1 to 12, wherein the preservation solution is the aqueous solution further containing a glycosaminoglycan.

14. The preservation method according to claim 13, wherein the concentration of the glycosaminoglycan is 0.1% (w/v) or more and 10% (w/v) or less.

15. The preservation method according to claim 13 or 14, wherein the glycosaminoglycan includes one or more glycosaminoglycans selected from the group consisting of chondroitin sulfate and hyaluronic acid.

16. A preservation solution having a potassium ion concentration of more than 0 mM and less than 115 mM, for preserving neural tissue derived from pluripotent stem cells at a preservation temperature of 8°C to 30°C.

17. The preservation solution according to claim 16, wherein the preservation temperature is 12°C to 22°C.

18. The preservation solution according to claim 16 or 17, wherein the neural tissue is a cell aggregate having a layer structure.

19. The preservation solution according to claim 18, wherein the neural tissue is a cell aggregate having a neuroepithelial structure.

20. The preservation solution according to claim 19, wherein the neuroepithelial structure is a continuous epithelium structure present on a surface of the cell aggregate and/or a rosette structure present on a surface of or inside the cell aggregate.

21. A transport method for neural tissue, comprising transporting neural tissue in a preservation solution having a potassium ion concentration of more than 0 mM and less than 115 mM at a preservation temperature of 8°C to 30°C.

22. The transport method according to claim 21, wherein the preservation temperature is 12°C to 22°C.

23. The transport method according to claim 21 or 22, wherein the neural tissue is a tissue derived from human pluripotent stem cells.

24. The transport method according to any one of claims 21 to 23, wherein the neural tissue is a tissue to be subjected to transplantation.

25. The transport method according to any one of claims 21 to 24, wherein the neural tissue is a cell aggregate having a layer structure.

26. The transport method according to claim 25, wherein the neural tissue is a cell aggregate having a neuroepithelial structure.

27. The transport method according to claim 26, wherein the neuroepithelial structure is a continuous epithelium structure present on a surface of the cell aggregate and/or a rosette structure present on a surface of or inside the cell aggregate.

28. The transport method according to any one of claims 21 to 27, wherein the neural tissue is one or more neural tissues selected from the group consisting of forebrain tissue, midbrain tissue and retinal tissue

29. A composition for transplantation at a temperature of 12°C to 22°C, comprising an aqueous solution having a potassium ion concentration of more than 0 mM and less than 115 mM and pH controlled by a buffering agent within the range of 6.0 to 8.6 and a human neural tissue derived from pluripotent stem cells.

30. The composition according to claim 29, wherein the neural tissue is a cell aggregate having a layer-like neuroepithelial structure.

31. The composition according to claim 30, wherein the neuroepithelial structure is a continuous epithelium structure present on a surface of the cell aggregate and/or a rosette structure present on a surface of or inside the cell aggregate.

32. The composition according to any one of claims 29 to 31, wherein the neural tissue includes one or more neural tissues selected from the group consisting of forebrain tissue, midbrain tissue and retinal tissue.

33. The composition according to any one of claims 29 to 32, wherein the aqueous solution is the aqueous solution further containing a glycosaminoglycan of 0.1% (w/v) or more and 10% (w/v) or less.
